# EUROPEAN PATENT APPLICATION

(11) **EP 2 399 507 A1**
(43) Date of publication of application: **28.12.2011**
(21) Application number: 11171132.1
(22) Date of filing: 23.06.2011
(51) Int. Cl.: A61B 1/01, A61B 18/26, G02B 6/44, H01S 3/067, A61B 1/00

(54) **Fibers and tips thereof used with devices**

(30) Priority: 23.06.2010 US 822027
(71) Applicant: Lumenis Ltd., 20692 Yokneam (IL)
(72) Inventor: Lewinsky, Reuven M., 36005 Israel (IL)
(74) Representative: Bardo, Julian Eason

(57) **Abstract**

Disclosed are apparatus, systems, devices, methods and structures, including an apparatus that includes a device including an inner channel, and a fiber structure comprising an energy-transmitting fiber and a cover surrounding the energy-transmitting fiber, the energy transmitting fiber including an end that is substantially flush with the cover such that the fiber structure can be passed through the inner channel of the devise.

## Description

### CROSS-REFERENCE TO RELATED APPLICATIONS

This application is a continuation-in-part application of non-provisional U.S. patent application serial No. 12/417,272, entitled "Multi-Stage Procedures with a Modifiable Fiber and a Guide Tube Containing an Instrument" and filed April 2, 2009, which in turn claims priority to provisional U.S. application Serial No. 61/044,371, entitled "Method and Apparatus for Laser Assisted Endoscopic Management," filed April 11, 2008, and to provisional U.S. application Serial No. 61/053,884, entitled "Device and Method for Endoscopic Interventions," filed May 16, 2008, the contents of all of these applications are hereby incorporated by reference in their entireties.

### BACKGROUND

Fibers to deliver radiation (e.g., laser radiation) are used in many fields, including the field of medicine where the use of laser radiation to facilitate performing surgical procedures has become prevalent.

For example, ERCP (Endoscopic Retrograde Cholangio-Pancreatography) is a diagnostic and therapeutic endoscopic procedure in Gastroenterology. It is used to diagnose and treat bile duct obstruction which is most commonly caused by bile duct stones and less frequently by tumors. ERCP is typically a two stage procedure where first the opening of the bile duct into the small intestine has to be widened by an incision (sphincterotomy), typically performed with an electrical wire (sphicterotome), followed by removal of the bile stones with a balloon catheter or a basket, or by breaking the stones (lithotripsy) which can be done by mechanical means (basket) or by application of energy using an EHL-based procedure (electro-hydraulic lithotripsy) or using laser. Other medical procedures that are predicated on the application of radiation include laser lithotripsy performed to remove urinary tract stones, etc.

When using laser radiation to perform the obstruction removal operation, such a procedure should be done under direct visualization to ensure maximal safety and prevent damage to adjacent tissue, especially the wall of the common bile duct ("CBD"). Direct visualization can be achieved using a choledochoscope (also referred to as a "baby scope") which provides an image of the inner cavity and contents of the CBD. The choledochoscope sometimes has to be passed through the working channel of the duodenoscope (also referred to as a "mother scope") and then be directed into the CBD. Bending at an angle as sharp as 90⁰ may be required to divert the choledochoscope. To achieve such bending, one or more steering mechanisms may be used, e.g., a moving metal tooth, commonly referred to as the "elevator", that is embedded at the end of the mother scope. When applied to bend the choledochoscope, such steering mechanisms often cause damage to its fiber-optic components resulting in loss of image. These devices are very expensive and the damage is irreparable.

An alternative technique that has been suggested to control the movement of the baby scope is to use a guide-wire that is inserted into the working channel of the duodenoscope. The guide-wire can be bent by the elevator and directed, for example, into the CBD and subsequently the baby scope is passed over the guide wire to follow it into the CBD. Once the choledochoscope is positioned well within the CBD, the guide-wire is pulled back and out of the working channel of the choledochoscope, and a fiber to transmit laser energy from a laser device to the bile stone has to be inserted through the same working channel until it protrudes out of the choledochoscope and close to the stone.

Passing the laser fiber is often difficult, sometimes impossible, and due to its sharp tip and high stiffness, it may damage the choledochoscope. Such damage most commonly occurs close to the distal part where the choledochoscope is already bent at its exit from the duodenoscope.

### SUMMARY

The present disclosure relates to a modifiable laser fiber structure adapted to perform at least one function (e.g., side-firing of laser radiation) when operated in one configuration, and to perform at least another function (e.g., straight-firing) when the laser fiber structure is modified (e.g., structurally modified) to operate in another of its configurations.

The present disclosure also relates to methods, apparatus and devices to perform multi-stage procedures, such as multi-stage surgical procedures.

For example, the present disclosure relates to performing surgical procedures with a modifiable laser fiber structure that can operate in at least two configurations. In some embodiments, the laser fiber is placed into a body of a patient and is operated in an initial configuration (e.g., sideways emissions to perform incisions to widen the opening of the bile duct, a procedure referred to as sphincterotomy). Subsequently, the laser fiber structure can be modified (e.g., cutting or decapitating the tip of the fiber that enabled the sideways emission operations) so that it is adapted to operate in another configuration (e.g., straight direction emissions). In some embodiments, the sequence of configurations can be reversed (e.g., the fiber may be configured to first enable straight-shooting emissions, and then be modified to perform sideways emissions).

The laser fiber structure can thus be used to perform a procedure where initially a side firing fiber is passed through the working channel of a scope (e.g., duodenoscope or some other "mother scope") to perform the sphincterotomy. In some embodiments, the fiber may then be pulled out of the scope, and its tip removed. Subsequently, the laser fiber structure is reconfigured to operate as a straight firing laser fiber structure. The fiber may then be passed through the working channel of another scope (e.g., a choledochoscope, or some other "baby scope") which itself has been passed through the working channel of the mother scope and can now be used for bile lithotripsy.

Thus, in some embodiments, the apparatus, devices and methods described herein may be used to perform laser assisted ERCP (sphincterotomy + lithotripsy).

The modifiable fiber structure may also be used to perform other types of medical procedures, e.g., urinary or pulmonary tract obstruction removal and/or procedures in which scopes holding a fiber structure such as the one described herein are directed through tubular anatomical structures to operate the modifiable fiber structure in one or more if its various configurations, as well as procedures relating to other fields (e.g., industrial application procedures).

The present disclosure also describes apparatus, devices and methods which provide a solution to difficulties commonly encountered during performance of procedures based on the use of scopes holding treatment instruments (e.g., performance of laser bile lithotripsy). A hollow guide-tube facilitates moving a baby scope (e.g., choledochoscope) into a position near an area to be treated (e.g., into the CBD), practically serving as a guide wire. The guide tube also facilitates advancing an instrument, such as a laser fiber, contained within the guide tube through the working channel of a baby scope to the position near the area to be treated, by preventing sticking of the sharp fiber tip into the wall of the working channel of the choledochoscope and by reducing friction between the fiber and the same.

Also described herein are apparatus and methods for performing treatment procedures, including medical treatment procedures such as laser-assisted bile lithotripsy as part of an ERCP. The laser fiber (or some other instrument or tool) is preloaded into the guide-tube until the tip of the instrument is positioned approximately several centimeters from the distal tip of the guide tube. This arrangement of the guide tube and instrument element is then inserted into the working channel of a scope, such as the choledochoscope. In some embodiments, the fiber, guide tube and choledochoscope are inserted into another scope (i.e., the mother scope), such as a duodenoscope, and pushed to a position near a steering mechanism generally configured to steer the system or device in the working channel of the other scope (e.g., to steer/guide the scope containing the guide tube and instrument arrangement). Such a steering mechanism may include a pivotable elevator of the duodenoscope. The guide-tube is pushed out of the duodenoscope and directed into the CBD with the help of the elevator, if required. The elevator is then retracted into its nonbent (lying) position and the choledochoscope (or some other baby scope) is advanced over the guide-tube into the CBD until it passes the guide-tube which is now inside the advancing scope. Once the baby scope (e.g., choledonchoscope) is in proximity to the target area to be treated, e.g., an obstruction such as a stone that is to be removed, the laser fiber is further advanced such that it protrudes out of the scope, and laser lithotripsy may then be performed.

In some embodiments, devices and/or apparatus having other configurations and manufactured using different materials may be used.

In one aspect, an apparatus is disclosed. The apparatus includes a modifiable fiber structure adapted to operate in at least two configurations such that in a sideways-firing configuration the fiber structure is adapted to emit radiation in a substantially sideways direction relative to a longitudinal axis of the fiber structure, and in another, axial-firing, configuration the fiber structure is adapted to emit radiation in a substantially axial direction relative to the longitudinal axis of the fiber structure.

Embodiments of the apparatus may include one or more of the following features.

The apparatus may further include a scope to receive the fiber structure when the fiber structure is adapted to operate in the sideways-firing configuration, and another scope to receive the fiber structure when the fiber structure is adapted to operate in the axial-firing configuration.

The modifiable fiber structure may be adapted to operate in the sideways-firing configuration may be adapted to emit radiation in the substantially sideways direction to widen the opening of at least a bile duct of a patient.

The fiber structure adapted to operate in the axial-firing configuration may be adapted to operate in the axial-firing configuration to emit radiation in the substantially axial direction to remove an obstruction in at least a bile duct.

The fiber structure may include a radiation-transmitting fiber coupled to a cap. In the other configuration of the fiber structure the cap of the fiber structure may be removed to uncover a portion of the radiation transmitting fiber.

The apparatus may further include a radiation source to generate radiation, the radiation source optically coupled to the fiber structure such that generated radiation is transmitted from the radiation source to the fiber structure.

In another aspect, another apparatus is disclosed. The other apparatus includes a guide tube disposed within a scope, the guide tube configured to be advanced to a position near an area to be treated so that the scope can be advanced along the guide tube to the position near the area to be treated, The guide tube is further configured to receive a treatment instrument to perform a treatment procedure on the area to be treated.

Embodiments of the other apparatus may include any of the features of the first apparatus described above, as well as one or more of the following features.

The apparatus may further include the treatment instrument to perform the treatment procedure.

The treatment instrument may be a fiber to irradiate the area to be treated.

The fiber may include a modifiable fiber structure adapted to operate in at least two configurations such that in a sideways-firing configuration the fiber structure is adapted to emit radiation in a substantially sideways direction relative to a longitudinal axis of the fiber structure, and in another, axial-firing, configuration the fiber structure is adapted to emit radiation in a substantially axial direction relative to the longitudinal axis of the fiber structure.

The apparatus may further include a laser source to generate laser radiation, the laser source being coupled to the fiber.

The apparatus may further include another scope to receive the scope containing the guide tube, the scope containing the guide tube being positioned in the other scope such that the guide tube and the scope containing the guide tube may be advanced from an opening in a distal end of the other scope to the position near the area to be treated. The other scope may include a duodenoscope. The scope containing the guide tube may include a choledochoscope.

The guide tube may include a first segment extending from a proximal end of the guide tube towards the distal end of the guide tube, and a second segment located near the distal end of the guide tube. The treatment instrument may be disposed within the guide tube such that it does not occupy an inner lumen defined by the second segment prior to being advanced to the area to be treated. The treatment instrument may be configured to be advanced through an opening at the distal end of the guide tube at the area to be treated such that at least part of the treatment instrument extends outside the distal end of the guide tube.

The apparatus may further include a steering mechanism to facilitate steering the guide tube to the position near the area to be treated. The steering mechanism may include a pivotable elevator tooth to actuate at least the guide tube.

In yet another aspect, a method to perform multi-stage procedures is disclosed. The method includes directing a modifiable fiber structure to a target area, the modifiable fiber structure adapted, in a sideways-firing configuration, to emit radiation in a substantially sideways direction relative to a longitudinal axis of the fiber structure and further adapted in another, straight-firing, configuration to emit radiation in a substantially axial direction relative to the longitudinal axis of the fiber structure. The method also includes operating the modifiable fiber structure in at least the sideways-firing configuration and the straight-firing configuration.

Embodiments of the method may include any of the features described above in relation to the various apparatuses, as well as any of the following features.

Operating the modifiable fiber structure may include causing the modifiable fiber structure to emit radiation in the substantially sideway direction at the target area.

The method may further include modifying the modifiable fiber structure so that the modified fiber structure is adapted to operate in the straight-firing configuration.

Operating the modifiable fiber structure in at least the sideways-firing configuration and the straight-firing configuration may include coupling radiation generated by a radiation source to the fiber structure to cause emission of the radiation in one of the sideways-firing configuration and the straight-firing configuration.

In a further aspect, another method to perform multi-stage procedures is disclosed. The method includes placing a modifiable fiber structure adapted to operate in at least two configurations into a body of a patient, performing an initial stage of a procedure with the fiber structure operating in an initial configuration of the at least two configurations, and modifying the fiber structure so that the fiber structure is adapted to operate in another configuration of the at least two configurations.

Embodiments of the method may include any of the features described above in relation to the various apparatuses and method, as well as any of the following features.

Performing the initial stage of the procedure with the modifiable fiber structure operating in the initial configuration may include coupling radiation to the fiber structure to cause the fiber structure to emit radiation in a substantially sideways direction relative to a longitudinal axis of the modifiable fiber structure to cause an incision of an opening of at least a bile duct of a patient.

The method may further include performing another stage of the surgical procedure with the fiber structure operating in the other configuration of the at least two configurations. Performing the other stage of the procedure with the fiber structure operating in the other configuration may include coupling radiation to the modifiable fiber structure to cause the fiber structure to emit the radiation in a substantially axial direction relative to a longitudinal axis of the modifiable fiber structure to remove at least one or more bile duct obstructions.

Placing the modifiable fiber structure may include inserting the fiber structure through a scope of an apparatus having multiple scopes.

In an additional aspect, a treatment method is disclosed. The method includes inserting a guide tube containing a treatment instrument into a scope, the guide tube having a distal end with an opening, and advancing the guide tube to a position near an area to be treated so that the scope can be advanced along the guide tube to the position of the area to be treated.

Embodiments of the method may include any of the features described above in relation to the various apparatuses and the methods, as well as any of the following features.

The method may further include advancing the scope to the treatment area to substantially cover the guide tube, and advancing the treatment instrument through the opening at the distal end of the guide. Advancing the treatment instrument may include advancing the instrument through the opening at the distal end of the guide tube such that at least part of the treatment instrument extends outside the distal end of the guide tube and the scope.

The method may further include advancing the guide tube containing the treatment instrument through an opening in another scope, and advancing the scope having the guide tube through the opening of the other scope to substantially cover the guide tube advanced through the opening of the other scope.

The method may further include irradiating the area to be treated with laser radiation generated by a laser device and directed through the instrument, the instrument including a laser fiber.

The method may further include actuating a pivotable elevator tooth to facilitate steering the guide tube.

In another aspect, a further apparatus is disclosed. The further apparatus includes a moveable scope having a tubular body section, and an inflatable balloon disposed on at least a portion of the body section, the balloon configured to control movement of the scope when the balloon is inflated.

Embodiments of the apparatus may include any of the features described above in relation to the various apparatuses and various methods, as well as any of the following features.

The balloon, when inflated, may be configured to perform one or more of, for example, restrict movement of the scope, center the scope in a tubular organ of a patient's body and/or stabilize the scope in a substantially stationary position within the tubular organ of the patient's body.

The apparatus may further include a syringe containing fluid, and a tube connected at one end to the syringe and at another end to an opening of the balloon. The syringe may be configured to perform one or more of, for example, push the fluid into the balloon via the tube to inflate the balloon and/or withdraw fluid from the balloon.

In another aspect, an apparatus is disclosed. The apparatus includes a device including an inner channel, and a fiber structure comprising an energy-transmitting fiber and a cover surrounding the energy-transmitting fiber, the energy transmitting fiber including an end that is substantially flush with the cover such that the fiber structure can be passed through the inner channel of the device.

Embodiments of the apparatus may include any of the features described above in relations to other apparatus, systems, methods, devices, structures, and tools, as well as any of the following features.

The energy-transmitting fiber may include an optical fiber.

The fiber structure that can be passed through the inner channel of the device may be configured to be guided naturally through the inner channel of the device without using guiding mechanisms, the guiding mechanisms including a guide wire.

The device including the inner channel may include a scope-based device. The fiber may be no more than about 2 mm in diameter smaller than a working channel of the scope-based device.

The scope-based device may include a mother scope-baby scope endoscopic system that includes a mother scope including a working channel, and a baby scope fitting within the working channel of the mother scope. The mother scope may include a duodenoscope. The baby scope may include a choledocoscope.

The scope-based device may include a single scope-based system.

The apparatus may further include a laser subsystem optically coupleable to the energy-transmitting fiber.

In yet another aspect, a method is disclosed. The method includes inserting a fiber structure comprising an energy-transmitting fiber and a cover surrounding the energy-transmitting fiber into a device including an inner channel, the energy-transmitting fiber including an end that is substantially flush with the cover such that the fiber structure can be passed through the inner channel of the device. The method also includes advancing the fiber structure through the inner channel of the device.

Embodiments of the method may include any of the features described above in relations to other apparatus, systems, methods, devices, structures, and tools, as well as any of the following features.

The device may be a mother scope-baby scope system, and the method may further include inserting a mother scope and a baby scope of the mother scope-baby scope system into a patient's body, guiding the baby scope so that the baby scope's end is pointing in an appropriate direction for application of energy to achieve a desired therapeutic purpose, directing the fiber structure including the fiber with the substantially flush end through the baby scope's working channel without using a guide wire, and coupling optical energy through the fiber, the optical energy being emitted from the substantially flush end of the fiber towards a target.

The device may include a scope based device including one of, for example, a single scope-based system, and/or a mother scope-baby scope system.

The method may further include optically coupling a laser subsystem to the energy-transmitting fiber.

In a further aspect, a fiber structure is disclosed. The fiber structure includes an energy-transmitting fiber, and a cover surrounding the energy-transmitting fiber, the energy transmitting fiber including an end that is substantially flush with the cover such that the fiber structure can be passed through an inner channel of a device to receive the fiber structure.

Embodiments of the fiber structure may include any of the features described above in relations to other apparatus, systems, methods, devices, structure and tools, as well as any of the following features.

The energy transmitting fiber may be optically coupleable to a laser subsystem.

Details of one or more implementations are set forth in the accompanying drawings and in the description below. Further features, aspects, and advantages will become apparent from the description, the drawings, and the claims.

### BRIEF DESCRIPTION OF THE DRAWINGS

FIGS. 1A and 1B are schematic diagram of a modifiable fiber structure having at least two configurations (one configuration shown in FIG. 1A and another configuration shown in FIG. 1B).
FIG. 2 is a schematic diagram of another implementation of a modifiable fiber structure adapted to be operated in separate configurations.
FIG. 3 is a flowchart of a multi-stage procedure performed using a modifiable fiber structure.
FIGS. 4A-B are diagrams depicting a multi-stage medical procedure performed using a modifiable fiber structure.
FIG. 5 is a view of an apparatus including a guide tube and instrument arrangement to perform treatment procedures.
FIGS. 6A-B are partial views of the guide tube of FIG. 5.
FIG. 7 is a cross-sectional view of a guide tube, laser fiber and a baby scope.
FIG. 8 is a cross-sectional partial view of a guide tube, laser fiber and baby scope inside of a mother scope.
FIG. 9 is a partial view of a guide tube extending from a mother scope.
FIGS. 10A-B are partial views of a baby scope enveloping the guide tube extending from a mother scope.
FIG. 11 is a partial view of an arrangement of a laser fiber extending from a baby scope that extends from a mother scope.
FIG. 12 is a flowchart of a treatment procedure performed using, for example, the apparatus of FIGS. 5-7.
FIGS. 13A-G are diagrams of an apparatus used to perform a medical procedure.
FIG. 14 is a photograph of a laser shifter to facilitate displacing laser fibers.
FIGS. 15A-B are photographs of a scope-based device that includes an inflateable balloon.
FIG. 15C is a schematic diagram of a scope-based device that includes an inflateable balloon.
FIGS. 16a-c are schematic diagrams of the end portions of fiber structures, including fiber structures with substantially flush fiber ends.
FIG. 17 is a flowchart of a procedure using a fiber structure with a substantially flush fiber end.
FIG. 18 is a schematic diagram of an apparatus including a fiber structure with a substantially flush fiber end used to perform an obstruction removal procedure.
FIG. 19 is a photograph depicting apparatus used to test several configurations of fiber structures with substantially flush fiber ends.

### DETAILED DESCRIPTION

Disclosed are apparatus and methods to perform multiple operations, such as multi-stage procedures (e.g., surgical procedure involving several stages). In some embodiments, an apparatus is provided that includes a modifiable fiber structure adapted to operate in at least two configurations. In one configuration, the fiber structure is adapted, for example, to emit radiation in a substantially sideways direction relative to a longitudinal axis of the fiber structure, and in another configuration the fiber structure is adapted to emit radiation in a substantially axial direction (e.g., substantially parallel direction relative to the longitudinal axis) of the fiber structure. To operate in its other configuration, the fiber structure is modified (e.g., a portion of the fiber structure is physically altered) to render the fiber structure operational in the other configuration. The fiber structure may be modified to operate in additional configurations and may be adapted to operate in a different order of configurations (e.g., first operate in a straight-firing configuration, then modified to a sideways-firing configuration, and, in some embodiments, modified to again be configured to emit radiation in a straight-firing direction).

Also disclosed herein are apparatus and methods that can be used to treat a target area. In some embodiments, an apparatus configured to perform a treatment procedure (e.g., a surgical procedure) includes a double-function guide-tube to improve laser-assisted bile lithotripsy as part of an ERCP procedure. The outer surface of the guide-tube acts as a guide-wire on which a scope, such as a choledochoscope (baby scope) is slid and passively guided towards the target area (e.g., into the common bile duct).

The inner lumen of the guide-tube defines a passageway through which a treatment instrument, such as a laser fiber, may be inserted and advanced therein. In conventional procedures using a baby scope, after a guide wire and a baby scope enter the bile duct, the guide wire is pulled out of the baby scope such that its working channel is now vacant to pass treatment devices such as a fiber to deliver radiation. Trying to insert the laser fiber when the baby scope is already positioned within the common bile duct once the guide wire is removed often results in damage to the working channel caused by the sharp and relatively hard tip of the laser fiber, or is difficult due to friction between the fiber and the working channel wall, especially at bending points.

In some embodiments, the guide-tube may have a two segment implementation. Most of the guide tube is relatively stiff, thus facilitating pushing the guide tube inside the scope. A relatively soft distal part, typically several centimeters in length, is used to locate the bile duct entrance and to penetrate into it. In some embodiments, being a tube rather than a wire, enables to achieve the difference in stiffness (or rigidity) by the insertion of the laser fiber into most of the length of the guide-tube while leaving the distal part empty and thus softer without having to manufacture guide tubes having regions of different stiffness within the guide-tube itself.

In some embodiments, the laser fiber is preloaded into the guide-tube and the guide-tube with the pre-loaded fiber is used to guide the baby scope into the target area (i.e., prior to deployment of the guide tube during performance of various procedures).

In some embodiments, the apparatus may be used for other types of medical endoscopic applications. These may include, among others, urinary laser lithotripsy, treatment of strictures in the bronchial tree, colon polypectomy, etc. In some of these applications, the hollow guide-tube may be used in a single scope procedure to enable guiding the scope into treatment position and facilitating the passage of the laser fiber through its working channel. In some embodiments, other types of instruments, tools and/or devices may be disposed and/or travel in the inner lumen of the guide tube so that those other instruments/tools/devices can be advanced to the area to be treated whereupon they can be activated and/or actuated to perform their functions. Such instrument/tools may include electrical needles, balloon catheters, etc.

In some embodiments, the apparatus described herein may be used for other, non-medical applications (e.g., industrial application).

Further disclosed are apparatus, systems, devices, structures and methods, including an apparatus comprising a device including an inner channel, and a fiber structure comprising an energy-transmitting element (also referred to as an energy-transmitting fiber, fiber core, or fiber proper) and a cover surrounding the energy-transmitting element, the energy transmitting element including an end that is substantially flush with the cover such that the fiber structure can be guided naturally through the channel of the device (e.g., a working channel of an endoscope).

### Modifiable Fibers

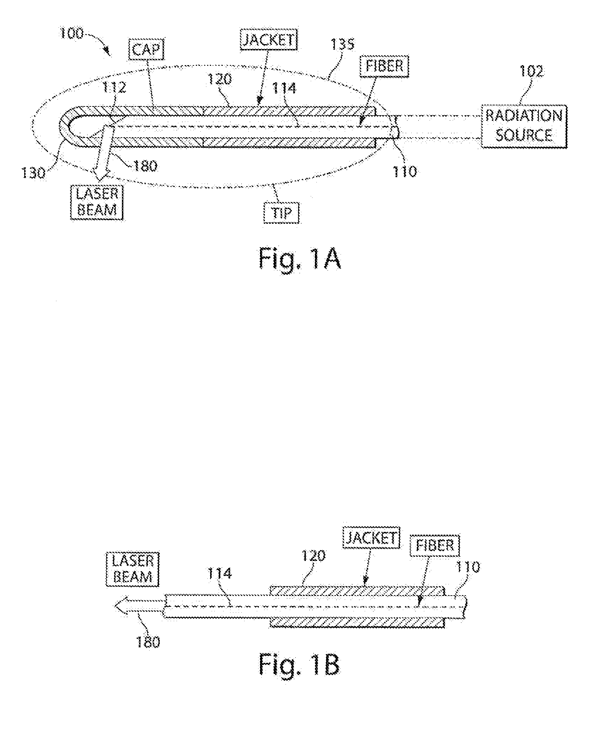

Referring to FIGS. 1A-B, schematic diagrams of a modifiable fiber structure 100 are shown. The modifiable fiber structure 100 is, in some embodiments, configured to transmit radiation to a target area to irradiate the target area (e.g., an organ or tissue) to achieve some therapeutic effect (e.g., tissue ablation, dissecting of the tissue, coagulation, etc.). The modifiable fiber structure includes a fiber 110, or some other radiation conduit or waveguide, to transmit radiation generated by a radiation source 102 (shown schematically in FIG. 1A). Suitable optical fibers may include fibers made from, for example, glass, plastic, fused silica, and any other type of suitable material. Suitable radiation sources may include, for example, Er:YAG laser systems, Ho:YAG laser systems and Nd:YAG laser systems. Other types of laser systems may be used.

In some embodiments, the fiber structure 100 may further include a surrounding layer, or jacket, 120 to shield the fiber 110 from physical damage (i.e., to preserve its structural integrity) and/or to further enable propagation of optical radiation through the fiber with minimal attenuation (e.g., the jacket 120 may be substantially opaque to prevent radiation to traveling in the fiber to be emitted to the surrounding).

As further shown in FIG. 1A, fitted on the end of the fiber through which radiation is be emitted to irradiate the target area is a cap 130. In some embodiments, the cap 130 is constructed from a substantially transparent material, such as, for example, fused silica. The cap 130 thus defines a shell into which the distal end of the fiber (i.e., the end through which the radiation is emitted) is received. In some embodiments, the distal end 112 of the fiber is slanted (skewed) at an angle, of, for example, 30-60° relative to a longitudinal axis 114 of the fiber 110. The slanted configuration of the fiber end can act like an opaque mirror provided that the refractive indices of the fiber and the gas inside the capillary are such that total internal reflection results. The resultant direction of the emitted beam is indicated by the arrow shown in FIG. 1A. The slanted configuration of the distal end 112 of the fiber 110 causes radiation emitted through that end to be emitted substantially at an angle based on the specific angle of the slanted end 112 and the indices of refraction of the fiber medium and medium surrounding the distal end 112 of the fiber 110.

Thus, in this configuration (i.e., with the slanted distal end 112 disposed within the cap 130), radiation, such as laser radiation, is emitted at an angle such that the emitted radiation is substantially in a sideways direction relative to the longitudinal axis 114 of the fiber 110. Thus, the fiber structure 100 is, in this configuration, a side-firing structure. In some embodiments, the angle at which the radiation is emitted is in the range of 70°-90°, depending, for example, on the slanted surface angle.

In the sideways-firing configuration the fiber structure may be used to perform a corresponding set of operations. For example, the fiber structure 100, in its side-firing (sideways-firing) configuration may be used to perform procedures requiring laser side-firing emissions. For example, the laser structure 100 may be placed near a tissue (e.g., the bile duct orifice) that needs to be cut. Cutting of the tissue is then achieved, for example, by laterally firing the radiation (e.g., laser radiation) emissions 180 in a substantially sideways direction relative to the longitudinal axis of the fiber structure.

The distal end section of the fiber structure 100, including the cap 130, at least a portion of the distal section of the fiber 110 (including the slanted end) and at least a portion of the distal section of the jacket 120, defines a tip 135 of the structure. As will become apparent below, to modify, or otherwise adjust the configuration of the fiber structure to cause it to perform other operation(s), the tip 135 is removed.

Specifically, and with reference to FIG. 1B, the fiber structure 100 may be modified, for example, to remove at least a part of the tip 135 to remove the cap, the slanted end 112 and a portion of the jacket 120 depicted in FIG. 1A. In some embodiments, the tip 135 is removed as a single unit by, for example, cutting the fiber approximately 20-70mm behind the tip (total decapitation). This type of structural modification will cause radiation traveling through the fiber 110 to exit the fiber 110 at an axial angle that is substantially parallel to longitudinal axis 114 of the fiber 100 (the actual exit angle of the radiation will depend, at least in part, on the indices of refraction of the fiber tip and the medium surrounding the end of the fiber 110). As shown in FIG. 1B, the radiation emissions 180 are emitted in an axial direction that is substantially parallel to the longitudinal axis 114 of the fiber structure 100.

Thus, in its modified configuration, the fiber structure 100 is configured to emit radiation in an axial orientation relative to the longitudinal axis of the fiber 110. In this radiation emission mode, the fiber structure 100 may be used to perform operations requiring axial-firing (also referred to as straight-firing) radiation emissions. For example, whereas the fiber structure 100 may have been used in its side-firing configuration to widen an opening to the bile duct, the same fiber structure 100 may be modified, after performing the bile duct opening operation, so that it is configured to emit radiation in a substantially axial direction relative to the longitudinal axis of the fiber to, for example, fire at an obstruction inside the bile duct (e.g., a bile-duct stone). By modifying a particular fiber structure so that it is can emit laser radiation in at least two different orientations to thus perform at least two different functions, separate fibers or instruments, each configured to perform only one set of operations (e.g., one fiber to perform side-firing, and another, different fiber to perform straight-firing operations) are not required.

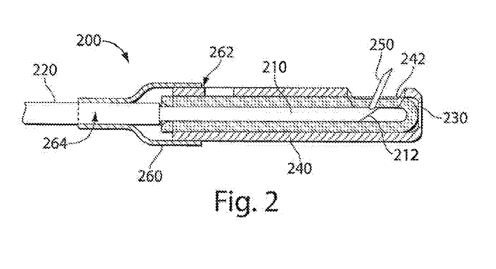

Referring to FIG. 2, a cross-sectional diagram of an implementation of a modifiable fiber structure 200 is shown. The fiber structure 200 is adapted to operate in at least two configurations. In one configuration, radiation is emitted from its distal end and is substantially sideways relative to a longitudinal axis of the fiber, and in another configuration (e.g., when the structure is modified through cutting the tip of the structure), the radiation is emitted in a substantially axial direction. The fiber structure 200 includes a substantially translucent or transparent cap 230 in which an optical fiber 210 is received. The fiber 210 may be similar to the fiber 110 of the fiber structure 100 depicted in FIGS. 1A-B. As with the fiber structure 100, the fiber 210 of the structure 200 has a slanted (skewed) distal end 212, relative to the longitudinal axis of the fiber, that is adapted to cause radiation emitted through the end to be emitted substantially at an angle (e.g., sideways angle) relative to the longitudinal axis of the fiber 210.

The cap 230 is fitted within a casing 240 that is substantially opaque so that radiation emitted through the distal end 212 of the fiber 210 is not emitted to the ambient surrounding of the fiber structure 200 except through a window defined in the casing. The casing 240 thus includes an opening, or a window, 242 that is defined proximate to the slanted end 212 of the fiber 210. Thus, radiation emitted through the slanted end 212 can exit the fiber structure 200 through the window 242. For example, as shown in FIG. 2, emitted radiation portion 250 (represented as a narrow pin protruding from the window 242) exits the window at a substantially sideways direction relative to the longitudinal axis of the fiber 210.

As further shown in FIG. 2, in some embodiments, the fiber structure 200 may include a tubular sleeve 260 having two open ends. The sleeve 260 is, in some embodiments, a polymeric shield configured to enable making the transition between the capillary, the metal cap and the fiber jacket smooth, and is also configured to protect the structure from breaking at that point by providing a strain-relief mechanism. The sleeve 260 receives through a first end 262 the end portion of the casing 240, and receives a jacket 220 (i.e., a cladding layer shielding the fiber 210) through its other open end 264. The diameter of the opening 262 and 264 are generally slightly smaller than the respective diameters of the casing 240 and jacket 220 so that the casing 240 and jacket 220 are snugly fitted within the sleeve 260 to keep the various components of the structure in place, i.e., through tension forces exerted by the walls of the sleeve 260 on the casing 240 and the jacket 220. The sleeve 260 thus enables mechanical connection of the various parts of the fiber structure 200.

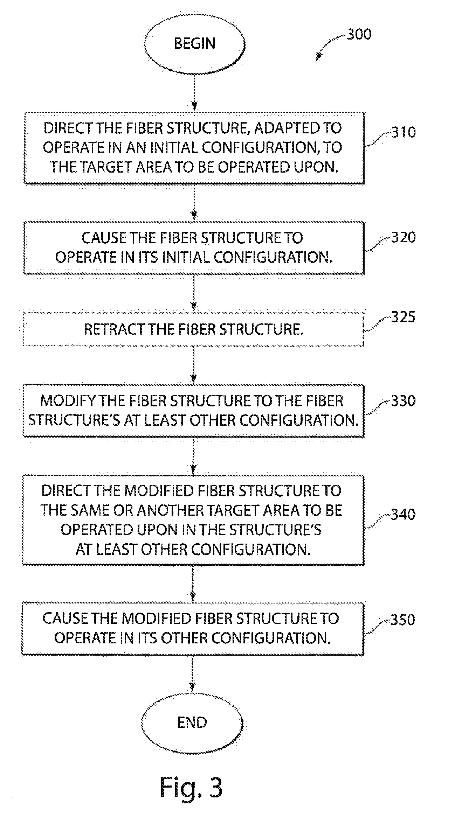

Referring to FIG. 3, a flowchart of a multi-stage procedure 300 performed using a modifiable fiber structure is shown. In operation, the modifiable fiber structure may initially be placed, when adapted to operate in one configuration (e.g., adapted to emit radiation in a sideways direction relative to the longitudinal axis of the fiber structure) within an instrument, such as a scope, that is adapted to direct the fiber structure to an area to be treated and to enable the fiber structure to operate in the fiber structure's initial configuration. For example, in some embodiments, the instrument may be a duodenoscope (mother scope) having an inner channel through which the fiber structure, such as the fiber structure 200, is passed.

The fiber structure, adapted to operate in its initial configuration, is directed 310, either via a scope or without a scope, to the target area upon which the fiber structure is to operate upon. For example, when used for medical applications, the fiber structure is guided to a particular location in a body of a patient, and upon reaching the target area or being positioned proximate to the target area, the fiber structure is caused to operate 320 in its initial configuration. For example, a laser source such as the source 102, generates radiation that is transmitted through the fiber and emitted at its distal end in a sideways direction. In circumstances in which the fiber structure is guided to the target area via a guiding scope such as a duodenoscope, an opening of the duodenoscope enables radiation emitted from the fiber of the fiber structure to reach the target area.

Subsequent to the operation performed in the structure's initial configuration, the fiber structure is modified 330 to its at least other configuration (e.g., the fiber structure is physically altered to transform it into its other configuration). For example, after operating in its sideways firing configuration, the scope containing the fiber structure may optionally be retracted 325, and the tip of a fiber structure (e.g., the fiber structures 200), including the cap and the slanted end of the fiber, is removed. Such a modification alters the fiber so that its end edge is substantially sideways (i.e., perpendicular) to the longitudinal axis of the structure. In this structural configuration, the fiber structure is adapted to emit radiation in a substantially straight direction (i.e., substantially axial direction relative to the longitudinal axis of the fiber structure). The modified fiber structure is then directed 340 to the same or other target area (in some embodiments, guiding of the fiber structure can be facilitated by a scope) and upon reaching the target area the modified fiber structure is caused to operate 350 in its other configuration (e.g., by transmitting and emitting radiation generated by a laser source).

As noted, in some embodiments, the modifiable fiber structure may be adapted to operate in a different order of configurations (e.g., first straight-firing, then sideways-firing) and may also be adapted to operate in additional configurations.

A modifiable fiber structure such as the fiber structures 200 described herein may be used with a variety of applications, including medical applications, industrial application, etc., and may be used to perform a number of functions in its various configurations, including, for example, cutting, ablating, etc.

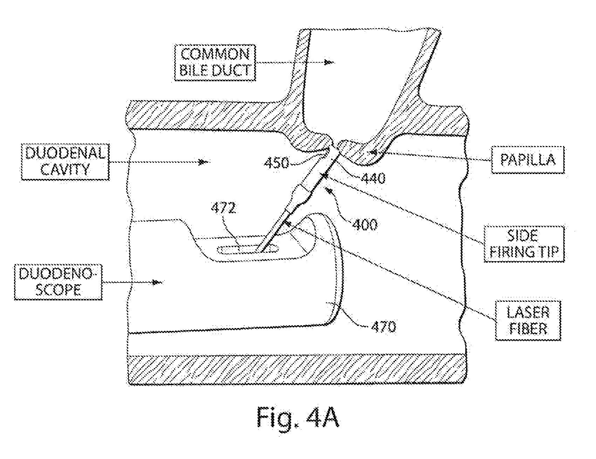

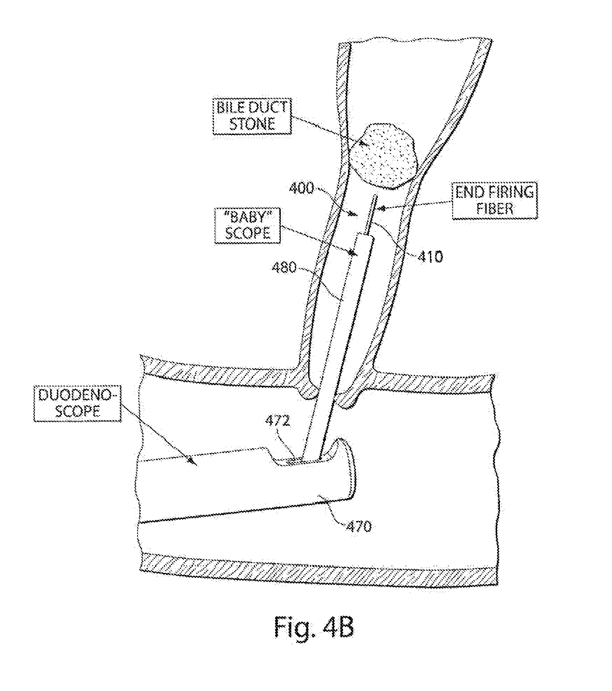

Referring to FIGS. 4A and 4B, diagrams of a fiber structure 400 used to perform a medical procedure are shown. The fiber structure 400, which may be similar to the fiber structure 100 and/or the fiber structure 200 described herein, may be utilized to perform a bile duct obstruction (e.g., stone) removal procedure. Other types of medical procedures in which a modifiable fiber structure is directed through tubular anatomical structures of the body to operate the modifiable fiber structure at a target tissue accessed through the tubular anatomical parts include procedures to open strictures in the upper or lower airways (trachea and bronchi) caused by tumors and/or procedures performed at the urinary tract that include opening strictures in the ureters and subsequently breaking urinary stones. In such procedures, operation of the modifiable fiber structure in its various configurations may enable an improved and better completion of the surgical procedure.

As previously noted, one type of a medical procedure in which the modifiable fiber structure may be used is a bile duct obstruction removal procedure. A bile duct obstruction procedure requires opening of a bile duct, followed by removal of the obstruction (e.g., a stone) located in the now open bile duct.

In some embodiments, a modifiable fiber structure maybe used to perform both operations, namely, the opening of the bile duct, and the removal of the obstruction. Specifically, and as shown in FIG. 4A, the fiber structure 400, adapted to operate in a sideways-firing configuration is guided to a location near the bile duct, for example, to a location near the papilla (i.e., the tissue surrounding the opening between the common bile duct and the duodenal cavity). In some embodiments, the fiber structure may be guided to the papilla of the common bile duct of a patient's body using a scope such as a duodenoscope 470 (e.g., a scope configured to pass through the duodenal cavity). Under those circumstances, the scope is guided to the location to be treated (in this case, the papilla near the opening of the common bile duct), and once the scope reaches its destination location, the fiber structure (which may have earlier been fitted within the inner hollow volume of the duodenoscope 470), is manipulated to cause the end to emerge from an opening 472 of the duodenoscope 470. Other types of scopes may be used to traverse through tubular anatomical structures in the body to reach intended target areas to be treated.

As further shown in FIG. 4A, in some embodiments, the fiber structure 400 includes, similarly to the fiber structure 100 and/or the fiber structure 200 shown in FIGS. 1A-B and FIG. 2, respectively, a translucent or transparent cap (not shown in FIGS. 4A or 4B) encasing a slanted end of a fiber through which radiation is guided from a radiation source to the target area to be treated. The slanted end of the fiber (which may be similar to the slanted end 212 of the fiber 210 of the fiber structure 200 shown in FIG. 2), in conjunction with the cap (similar to the cap 230 shown in FIG. 2), causes radiation to be emitted in a sideways direction relative to the longitudinal axis of the fiber structure 400 when laser radiation is coupled into the fiber. Radiation emitted through the slanted end of the fiber of the fiber structure 400 is directed through the translucent or transparent cap, and through an opening 442 of a casing 440 surrounding the fiber fitted within the cap (which may be similar to the casing 240 shown in FIG. 2).

Thus, in operation, the scope 470 is guided through, for example, the duodenal cavity, and its opening 472 is positioned near the papilla of the common bile duct. The fiber structure 400 is directed through the opening 472 of the scope 470 so that the opening 442 of the casing 440 of the fiber structure 400 is placed near the papilla surrounding the common bile duct. At this stage, the fiber structure is adapted to operate in its initial configuration, and accordingly, after the opening 442 of the casing 440 of the fiber structure 400 has been placed near the intended target area (the fiber structure may be spatially guided using a steering or guiding mechanism, such as, for example, the guide-tube-based system discussed in greater details below), the fiber structure is caused to fire sideways emissions by, for example, firing a radiation source (not shown in FIGS. 4A-B) coupled to the fiber structure 400. The sideways emitted radiation, marked as reference numeral 450 in FIG. 4A, is applied to the papilla to cause the tissue to be cut, thus opening, or widening an opening leading to the inner sections of the common bile duct.

Subsequently, the fiber structure 400 is modified so that it is adapted to operate in at least another configuration, in this case, a straight-firing configuration. Particularly, the fiber structure 400 is modified to, for example, remove (e.g., decapitate) the entire tip of the structure (the tip including part of the fiber, the cap and part of the casing) that caused the side firing operation. In some embodiments, to modify the fiber structure the fiber structure is retracted (the scope 470 may remain in its position near the target area, or it may also be retracted), and once retracted, the tip of the fiber structure 400 is cut, or clipped, to remove, for example, the casing 440, the cap covered by the casing 440, and the slanted end that was fitted within the cap (as was depicted in FIGS. 1A-B and 2). The physical alteration of the fiber structure results in a modified fiber structure in which the end portion of the fiber structure includes an uncovered fiber whose end has an edge that is substantially sideways (normal) to the longitudinal axis of the fiber structure 400. In that modified configuration, radiation (e.g., laser radiation) directed through the exposed fiber of the fiber structure is emitted in a substantially straight (axial) direction relative to the longitudinal axis of the fiber structure 400.

Thus, and as shown in FIG. 4B, depicting operations of the multi-stage procedure after modification of the fiber structure 400, an uncovered fiber section 410 of the fiber structure 400 is guided through the internal volume of the common bile duct to be positioned proximate to the bile duct obstruction to be removed (in this case, a bile duct stone). As noted, the fiber structure 400 and its fiber 410 are configured so that when radiation is directed through the fiber 410 (the radiation source is not shown in the figure), the radiation is emitted in a substantially axial direction relative to the longitudinal axis of the fiber structure 400 and/or the fiber 410. Thus, when radiation is directed through the fiber 410, the emitted radiation will be directed at the obstruction. In some embodiments, the applied radiation has sufficient fluence (typically 5-20 W of power) and attributes (e.g., wavelength) suitable to cause breaking or dissolving of the obstruction.

In some embodiments, the fiber structure (in the example shown in FIG. 4B, the fiber structure was modified to leave an uncovered fiber section) is fitted within the scope 470 or within another, different, scope 480 (a scope such as a choledochoscope) that may be guided through the scope used during the earlier stage of the procedure. The scope 480 may be configured to provide a user with a visual of the bile duct, including a visual of the obstruction/stone that is to be removed.

Use of another scope such as the scope 480 may also enable better maneuverability of the scope and the fiber contained therein to more accurately guide the fiber to its intended target area. That is, the scope 480 may include steering, vision and displacement mechanisms to guide the scope to an area near the target area. Thereafter, the uncovered fiber 410 may be deployed so as to be positioned near the stone or obstruction, whereupon radiation may be directed through the fiber 410 and emitted in a straight direction towards the obstruction.

In some embodiments, devices having other configurations (e.g., additional and/or different configurations) and manufactured using different materials may also be used. In some embodiments, other types of procedures may be performed using the modifiable fiber structure described herein.

### Fibers with Flush Tips

As described above, the modifiable fiber structure, such as the modifiable fiber structure 100 depicted in FIG. 1, is adapted to be modified (e.g., by decapitation of its tip) to cause the fiber to operate in its second configuration/mode (e.g., straight-firing mode). In some embodiments, the modified fiber structure has in the "straight-firing" mode a substantially (e.g., approximately or entirely) flush end such that the fiber (i.e., the energy-transmitting core element) is substantially flush relative to the cover surrounding the fiber. In addition to enabling the modified fiber structure to operate in its second operational configuration, having a flush end (or tip) also enables the fiber structure to be passed through within various devices such as scope-based devices (e.g., to move without using other instruments/tools/implements to guide or steer the movement of the fiber structure within an inner channel of such devices). Generally, the flush tip facilitates fiber passage through the inner channel (e.g., working channel) and also prevents damage to the channel walls by the sharp tip which is common in "stripped" fiber tips. The sharp tip is commonly the solid silica core which is also quite hard.

A fiber structure configuration that includes a fiber with a substantially flush end may be used with any type of fiber structure (e.g., non-modifiable structures adapted to operate in a single configuration/mode, modifiable fiber structures adapted to operate in multiple configurations/modes, etc.) In some embodiments, use of a flush fiber tip configuration may also be used with other waveguide configurations such as (but not limited to) hollow silica waveguides used to transmit CO2 laser energy, etc. In some implementations the fiber structure includes an energy transmitting element, e.g. solid silica core, (also referred to as a "fiber," "fiber core," "fiber proper") and a cover, sometimes referred to as a jacket, which may be an integral part of it. The fiber structure is sometimes referred to collectively as a fiber.

Thus, as described above in relation to the modifiable fiber structure, and as described in further details below, disclosed are systems, apparatus, structures and methods that include an apparatus comprising a device including an inner channel, and a fiber structure comprising an energy-transmitting fiber (e.g., an optical fiber) and a cover (or jacket) surrounding the energy-transmitting fiber. The energy transmitting fiber includes an end that is substantially flush with the cover such that the fiber structure can be guided naturally through the inner channel of the device (e.g., traverse the channel unguided without the use of guiding/steering mechanisms such as guide tubes/wires).

In some embodiments, the systems, apparatus, structures and methods described herein may be used with devices, such as scope-based devices and other device types, where there is a sharp bend in the direction of the device, and thus in the direction of a channel through which the fiber passes. Such bends may have varying degrees of sharpness (such degrees of sharpness may be defined by the radius of curvature, which may be specified as a range, e.g., about 0-30mm). Fiber structures in which the fiber (e.g., the optical fiber) is not substantially flush with the cover (i.e., the fiber end protrudes) can result in the protruding fiber end scraping the walls of the inner channel of device, thus impeding the movement of the fiber structure or even stopping progress of the movement of the fiber structure altogether. A protruding end of a fiber can also damage the device through which it passes by scraping and scratching the interior walls defining the inner channel of the device. In some embodiments, damage to the devices (e.g., scope-based devices) from the fiber ends can further be reduced by, for example, smoothing the edges of protruding ends of the fibers (i.e., structuring the edges to have a more rounded shape), fitting caps over the protruding ends, etc. An example of a cap that can be fitted over the flush end of a fiber through which radiation would be emitted (i.e., the distal end) is a rounded cap which may be constructed from materials, such as a fatty materials (biodegradable polymer, dense gel, etc.), which shrink or evaporate with the first set of laser shots (e.g., the cap evaporates after the first laser shot). Additionally, to reduce damage to the device through which the fiber structure passes, in some implementations, a cap, e.g., constructed from a soft polymer, can be fitted over the cover, and a cap removal mechanism (e.g., placed at the distal opening of the device through which the fiber structure emerges upon traversing the inner channel) can be used to remove the cap to thus provide an open path for radiation to be emitted from the fiber end.

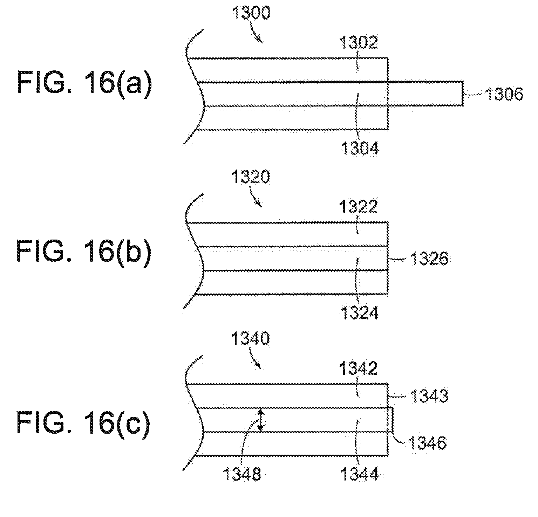

With reference to FIGS. 16a-c, schematic diagrams of end portions of fiber structures 1300, 1320 and 1340 are shown. FIG. 16a depicts the end of the fiber structure 1300 having a significant projection of a fiber 1304 beyond the end of a cover 1302 that surrounds the fiber 1302. As shown, the fiber 1304 has a protrusion 1306 extending beyond the end of the cover 1302. In some embodiments, such a protrusion 1306 has a length larger than the diameter of the fiber core or of the cover.

The cover 1302 surrounding the glass fiber proper 1304 is, in some embodiments, polymeric. A suitable material for the polymeric sheath may be a derivative of Teflon.

In contrast to the fiber structure 1300 depicted in FIG. 16a, the fiber structures described herein may have a substantially flush end in which there is only a minimal projection past the end of the jacket, as shown, for example, in FIG. 16b. That is, the fiber structure, such as the structure 1320, may be completely or approximately flush relative to the end of the cover, such as the cover 1322 (i.e., the fiber's end 1326 is flush with the end of the cover 1322).

Alternatively, in some embodiments, a fiber structure, such as the fiber structure 1340 of FIG. 16c, may have a small protrusion, e.g., a protrusion 1346, extending beyond the end of a cover 1342. A protrusion such as the protrusion 1346 extending slightly beyond the cover 1342 may be deliberately created (for example, for a structural or safety reason) or may be the byproduct of a more convenient or economical manufacturing process for the fiber structure that includes the fiber and the cover surrounding the fiber.

In some embodiments, the small protrusion 1346 may vary according to the desired application, the specific scope-based device being used and the bend radii encountered during such applications. In some embodiments, the extent by which the tip extends beyond the end of the cover (jacket) may be specified by the diameter of the fiber core, or the diameter of the cover surrounding the fiber (e.g., an example of specifying the extent of protrusion is to specify that the protrusion extends no more than a certain percentage, for example, 100% of an outer diameter d, marked 1348 in FIG. 16c, of the fiber core). In the above-described circumstances depicted in FIGS. 16b-c regarding the extent of the protrusion of the end of the fiber, the fiber end is considered to be substantially flush with the end of the cover of the fiber structure.

In some implementations, the fibers 1324 and/or 1344 include among others glass or crystalline fibers, Sapphire fibers, Germanate glass fibers, a combination of Germanate glass fibers with Sapphire tip, either in a solid core configuration or as a hollow waveguide, and/or other suitable fibers to deliver energy (e.g., laser energy). In some embodiments, fibers composed of Germanate glass fiber and fused silica tips may be used. Fibers used may include optical fibers adapted to transmit radiation (e.g., optical radiation) at various wavelength, including ultraviolet (UV) and infrared (IR) wavelengths.

The covers (jackets) 1322 and/or 1342 surrounding the fibers 1326 and 1346, respectively, may be constructed, in some embodiments, from polymeric materials such as, for example, poly(tetrafluoroethylene).

In some implementations, the fiber structure may vary based on the dimensions of the device's channel through which the fiber structure passes. For example, the diameter of a fiber with a cover/jacket may be of up to 2.5 mm smaller than that of the channel of the device through which the fiber structure passes. Other relative sizes for the fiber structures and inner channels of the devices through which the fiber structures may pass may be used.

A flush or close to flush end may be produced by a variety of operations. It may be produced, for example, by a cut of the jacketed fiber which leaves the end flush. Alternatively, it may be achieved by cutting off any protrusion. It may be produced by grinding off any protrusion and optionally polishing off or removing any material left at the end. Alternatively, a flush end may be produced by a cutting operation followed by grinding and/or polishing operations.

An apparatus comprising one or more devices and a fiber structure having a fiber with an end flush to the cover's end may further include any of a variety of appropriate radiation sources, including such radiation sources as laser devices. For example, a radiation source optically coupled to an optical fiber (e.g., using an optical coupler or adapter, an arrangement of optical elements, etc.) may include a laser subsystem such as, for example, an Er:YAG laser, a Ho:YAG laser, a Thulium:YAG fiber laser, a Nd:YAG laser, a frequency doubled double-pulse Nd:YAG (FREDDY) laser, Diode lasers including quantum-well lasers based, for example, on Antimonide (Sb) compounds such as In(Al)GaAsSb-based compounds, GaSb-based compounds, Thulium lasers and fiberlasers, etc. Other types of radiation sources (including optical radiation sources) may be used. Laser power delivered through an optical fiber may include radiation of 10 W or less, 20 W or less, 30 W or less, 100 W or less, and may also have other power levels.

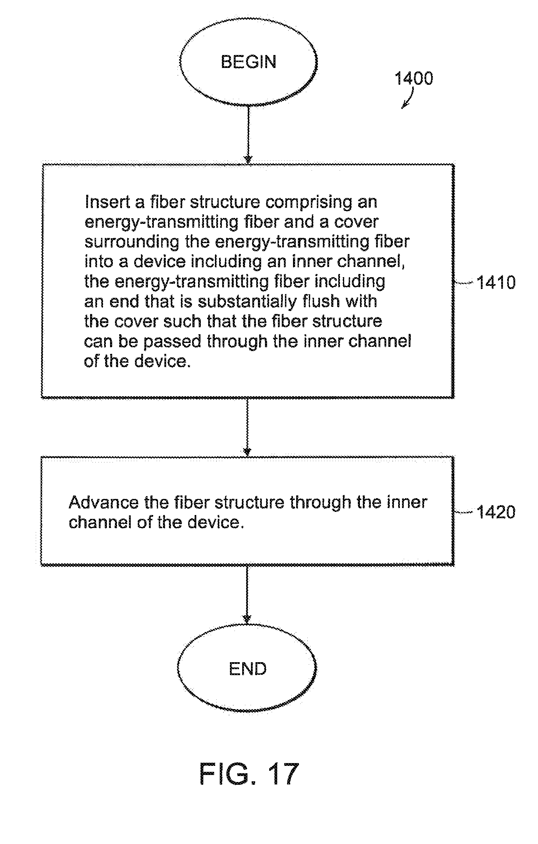

With reference to FIG. 17, a flowchart of a procedure 1400 using a fiber structure with a substantially flush fiber end is shown. A fiber structure, such as the fiber structures shown in FIGS. 16b-c, and which includes an energy-transmitting fiber and a cover surrounding the energy-transmitting fiber, is inserted 1410 into a device including an inner channel. The energy-transmitting fiber includes an end that is substantially flush with the cover such that the fiber structure can be guided naturally by the working channel of the device (i.e., passed unguided without having to use guiding or steering mechanisms such as guide wires). For example, the fiber structure can be passed through a device with an inner channel by pushing (e.g., by an operator) the fiber structure using moderate application of force and without guide tubes/wires or other implements that are conventionally used to direct a fiber structure through an inner channel of a device. Although the fiber structures described herein can be passed through the inner channel of a device without using guiding/steering mechanism (i.e., such fiber structures are guided naturally through the channel by, for example, moving along the walls of the channel), in some embodiments, a fiber structure with a flush end may be advanced through an inner channel using also a guiding/steering mechanism (e.g., a guide wire or guide tube) to further facilitate the advance of the fiber structure through the channel. In some embodiments, the device may include a scope-based device such as, for example, a mother scope-baby scope system, a single-scope system, etc.

Having inserted the fiber structure with the substantially flush fiber end, the fiber structure is advanced 1420 (e.g., by lightly pushing it) through the channel.

Advantageously, use of a fiber structure such as the fiber structures depicted in FIGS. 16b-c may save time and effort by, for example, avoiding having to use a guide tube to run the optical fiber past any bends with a narrow radius of curvature.

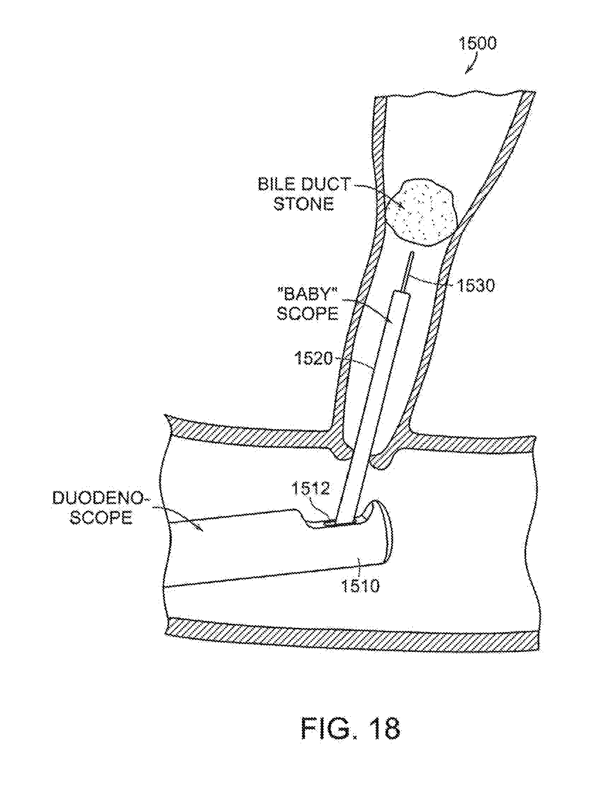

As noted herein, in some embodiments, a fiber structure with a flush tip may be used with scope-based devices. For example, a fiber structure with a flush tip, such as for example, the fiber structures 1320 and/or 1340 may be used with scope-based devices to perform bile stone removal. Thus, and with reference to FIG. 18 showing a schematic diagram of an apparatus 1500 to perform removal of an obstruction such as a bile stone, a duodenoscope 1510 is advanced in the duodenum to a position close to the bile duct. A baby scope 1520, such as a choledochoscope, is typically used first with an appropriate instrument to perform sphincterectomy to open space to reach the stone or other obstruction. The baby scope 1520 may be configured to provide a user with a visual image of the bile duct, including a visual image of the obstruction/stone that is to be removed. The baby scope 1520, emerging through the opening 1512 in the duodenoscope 1510, is then oriented and advanced in such a way that its distal end points toward the stone or other obstruction.

A fiber structure 1530, such as the structures 1320 and/or 1340 of FIGS. 16b-c, that includes an optical fiber (not specifically shown in FIG. 18) whose end is substantially flush with the cover of the fiber structure, is directed through the baby scope so that it is in place to apply energy (in this case laser energy) to the stone or obstruction. En route to the fiber's position where it applies energy to the obstruction, the fiber structure 1530 is naturally guided by the working channel (for example, by the channel's internal configuration), i.e., without using a guide wire or tube and without using other implements or tools (e.g., steering components such as an "elevator tooth" described in more details below) to guide the fiber structure. For example, in some embodiments, the fiber structure is advanced by applying moderate force on the fiber structure to push it through the channel of the scope. When the fiber structure is in position to have energy emitted from its substantially flush end towards the stone or obstruction, a laser system optically coupled to the fiber is activated and energy is applied to the stone or obstruction.

In some embodiments, the fiber structure with the flush fiber end may be used with single scope-based systems, such as single scope systems used in urology, bronchoscopy, etc., with other scope-based configuration, and with non-scope devices (e.g., shafts, tubular devices, etc.) A fiber structure with a flush fiber end may be used in a myriad of other medical and industrial applications.

To further illustrate the configurations, operation, and advantages of the apparatus, systems, devices, methods, and/or fiber structures described herein, tests involving fiber structures with substantially flush fiber tips were conducted. Particularly, the performance of two techniques to ameliorate the difficulties of inserting an optical fiber into a device (in this case scope-based device) were tested. The first technique was to polish the inserted end of the fiber flush with its jacket, reducing the sharp step of the silica. The second technique was the use of a guide tube for the fiber.

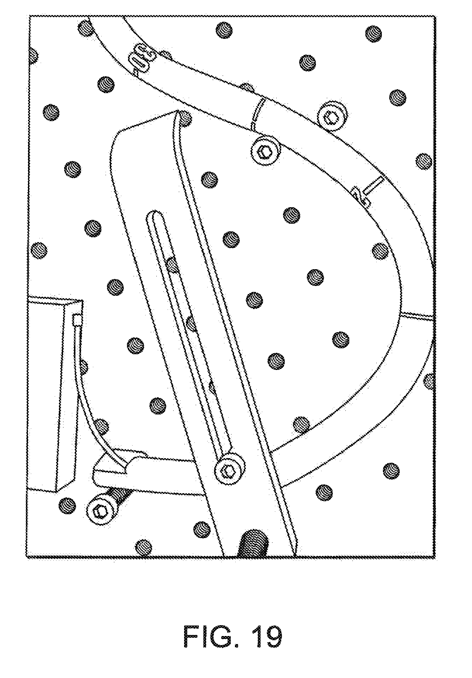

The device used in the experiment included was an Olympus scope (serving as a mother scope) that received another scope-based device (a baby scope), namely, a Lumenis Polyscope suitable for lithotripsy. The fibers used in the experiment were Lumenis SlimLine GI type, which are normally distributed with the fibers protruding out of their jackets, a configuration referred to as "stripped" fibers. The apparatus used in the experiment is shown in FIG. 19. The mother scope was laid out on a metal worktable, clamped between screws pointing upward from the work table, and its working end pointed toward a metal sheet perpendicular to the table. As shown, the baby scope's working end protrudes from the mother scope's working end. The distance between the perpendicular metal sheet and the end of the mother scope was adjustable to various values d. Four different values of this distance were tested, d = 6, 9, 12, and 18 mm. These distances can be used to define the angle of the bend through which the fiber structure was to pass.

Six fibers in all were tested. Three of them were of standard SlimLines GI type, and three were polished to have flush tips. For each given endoscope geometry all six fibers were tested (three times) to determine if they passed through (go/no go) using reasonable force. A guide tube was then inserted, and all six fibers were again tested to determine whether and how they passed through. The guide tube contained a fiber inserted up to 30 mm from the guide tube's end.

The results obtained without use of a guide tube are summarized in Table I:

**TABLE I**

| | d=6 mm | d=9 mm | d=12 mm | d=18 mm |
|---|---|---|---|---|
| Non-flush fiber 1 | - - - | - - - | - - - | + + + |
| Non-flush fiber 2 | - - - | - - - | - - - | + + + |
| Non-flush fiber 3 | - - - | - - - | - - - | + + + |
| Flush fiber 1 | + - + | + + + | + + + | + + + |
| Flush fiber 2 | + + + | + + + | + + + | + + + |
| Flush fiber 3 | + + + | + + + | + + + | + + + |

In this table, a plus sign "+" means that the fiber passed through, and a hyphen "-" means that the fiber did not pass through. As shown in table I, there are three plus or minus signs in the table entries to denote the three replications that were carried out with each fiber and each d value. As indicated, with the non-flush fibers at d=18mm, the fibers passed with great difficulty requiring a lot of force, and did not pass at all for smaller values of d. With the flush fibers, at d=6mm the fibers passed with difficulty requiring a lot of force. However, at d=9mm and above the flush fibers passed with mild application of force.

The results obtained with a guide tube are summarized in Table II:

**TABLE II**

| | d=6 mm | d=9 mm | d=12 mm | d=18 mm |
|---|---|---|---|---|
| Non-flush fiber 1 | + + + | + + + | NA | NA |
| Non-flush fiber 2 | + + + | + + + | NA | NA |
| Non-flush fiber 3 | + + + | + + + | NA | NA |
| Flush fiber 1 | + + + | + + + | NA | NA |
| Flush fiber 2 | + + + | + + + | NA | NA |
| Flush fiber 3 | + + + | + + + | NA | NA |

Here too, the symbols "+" an "-" have the meanings they had above. NA means that the experiment was not done because it was felt that its outcome could not be other than +.

As indicated in Table II, for both the non-flush and flush fibers, the guide tube with a fiber inserted up to 30 mm from its edge passed the scope easily starting at d=0 mm. At d=6 mm the tube passed smoothly, though trying to pull back the fiber and push it in again was difficult. This was true also for d= 9mm.

Thus, as indicated by the test results summarized in Tables I and II, polishing (or otherwise causing) the fiber end (tip) to be flush with its jacket improved the passing capabilities of the fiber structure through the Polyscope with and without a guide tube. Furthermore, when using a guide tube to pass a standard fiber through, the fiber bare edge carved through the inner side of the guide tube, and the debris accumulated in front of the fiber. That and the fact that the flushed fibers performed much better leads to the conclusion that one reason that movement of the fiber structure passing through a channel is impeded is because of a geometrical lock induced by the stripped fiber sharp edges.

### Guide Tube and Instrument Arrangements

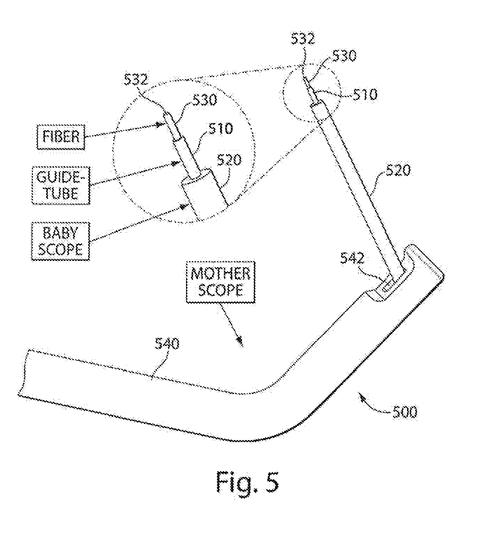

Referring to FIG. 5, a view of an apparatus 500 that includes a guide tube 510 disposed within a scope 520 (sometimes referred to as a baby scope) is shown. Disposed within the guide tube is a tool or instrument to perform operations on the target area to be treated. For example, the tool may be a fiber 530, such as an optical or laser fiber to deliver radiation from a radiation source (not shown in FIG. 5) coupled to the fiber. The fiber 530 may be, in some embodiments, a modifiable fiber structure, such as the modifiable fiber structure 200 described herein adapted to operate in at least two configurations. The radiation traveling in the waveguide 530 is then emitted at the tip 532 of the fiber 530 and applied to the target area (e.g., axial emission). The emitted radiation, e.g., laser radiation, can perform cutting functions, ablation functions, coagulation functions, etc. The guide tube 510 is configured to be advanced to the area to be treated. The scope 520 in which at least part of the guide tube 510 is disposed can then be advanced along the guide tube 510, which acts similarly to a guide wire, to approximately the location where the end of the guide tube is located. Advantageously, by advancing the flexible guide tube to the target location and then advancing the scope 520 along the guide tube 510 instead of having to use mechanical implements and guides, such as a pivotable elevator, to manipulate the scope, the scope 520 (which is typically constructed from expensive materials and parts) will not be subjected to excessive, and costly, wear and tear, thus enabling preservation of the structural integrity of the scope 520. Moreover, entering, for example, the CBD through the tiny papilla with the guide-tube is relatively easy compared to insertion of the baby scope on its own.

In some embodiments, the apparatus 500 includes a larger scope 540 (e.g., a mother scope), such as for example, a duodenoscope. As described herein, in some embodiments, the scope 520 or 540 may be a flexible endoscope (e.g., bronchoscope, urethroscope, etc.) that is typically passed through a natural orifice of the body (mouth, nose, rectum, vagina, urethra), or through an incision made on the body, to view the inside of the body, and to enable passing or attaching to the scope various instruments and tools to perform various operations.

The mother scope 540 includes, in some embodiments, a hollow flexible, or rigid, tube defining an inner "working" channel. The mother scope 540 includes an opening 542 located approximately at the distal end of the scope 540, through which tools/instruments, such as, for example, the laser fiber 530 (which may be disposed in the guide tube) and/or another scope (e.g., the baby scope 520) may be extended and deployed. Particularly, and as will become apparent below, when used to perform therapeutic/surgical procedures, the mother scope 540 may be advanced through the body and be positioned such that the opening 542 is positioned proximate the target area to be treated by the tool or instrument. The guide tube may be actuated or manipulated to be advanced to a position proximate the target area, and once the guide tube 510 reaches its destination location, the baby scope can be moved to a location near the target area by causing the baby scope 520 to be advanced along the guide tube 5 1 0.

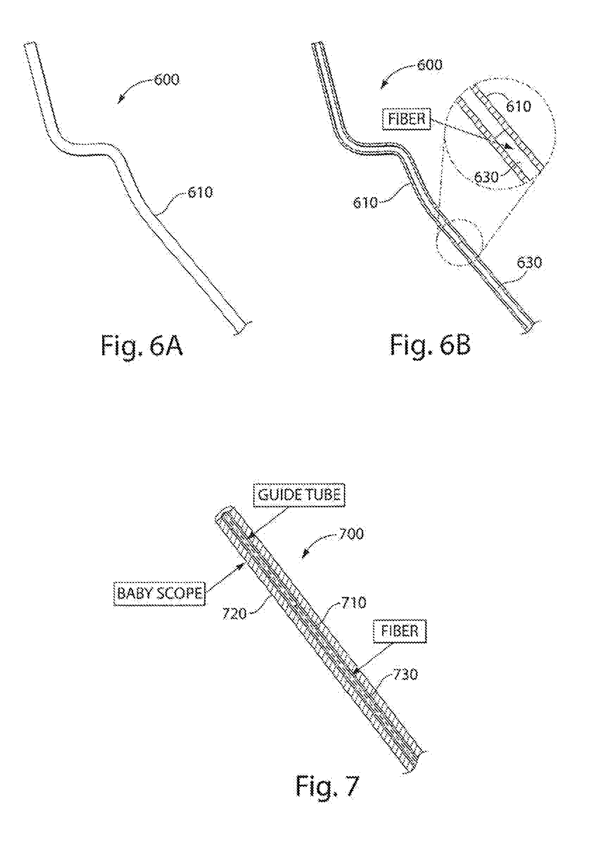

Referring to FIGS. 6A and 6B, diagrams of portions of a guide tube 610 and a waveguide, such as a laser fiber, 630, forming part of an apparatus 600 are shown. The guide tube 610 and the laser fiber 630 may be similar to the guide tube 510 and the waveguide 530, respectively, depicted in FIG. 5. As shown in FIG. 6B, providing a cross-sectional diagram of a portion of the guide-tube containing the laser fiber 630, the arrangement of the guide tube and laser fiber may be such that the laser fiber is partially inserted into the guide-tube so that the distal end of the laser fiber does not reach the distal end of the guide-tube 610. By partially inserting the laser fiber, the distal end of the guide-tube 610 retains its softness and flexibility. The section of the guide tube 610 that does not contain a segment of the laser fiber 630 can be contorted and/or otherwise manipulated to more easily advance the guide tube 610 in environments (e.g., inner parts of the body) where it may be difficult to advance scopes.

Referring to FIG. 7, a cross-sectional diagram of an apparatus 700 that includes a portion of a "baby" scope in which a guide tube 710 containing a portion of a laser fiber 730, is shown. Here too, the guide tube 710 may be similar to the guide tube 510 and/or the guide tube 610 depicted in FIGS. 5 and 6, respectively, while the laser fiber 730 may be similar to the laser fiber 530 and/or 630 depicted in FIGS. 5 and 6, respectively. In some embodiments, the scope 720 is advanced along the guide tube 710.

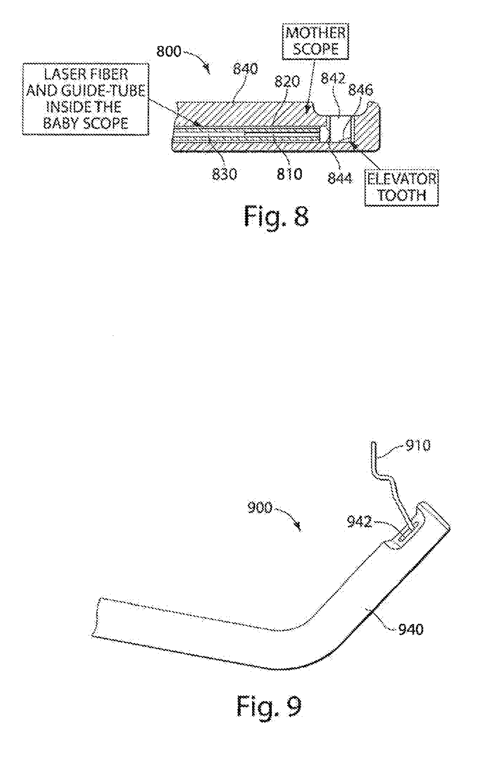

Referring to FIG. 8, a partial schematic cross sectional diagram of a scope-based apparatus 800 is shown. A baby scope 820, which may be similar to the scope 720 depicted in FIG. 7, includes an inner channel in which a guide tube 810 (which may be similar to any of the guide tubes 510, 610 and 710 depicted in FIGS. 5-7, respectively) containing a portion of a laser fiber 830. The scope 820 is placed in a working inner channel 844 of another ("mother") scope 840, such as a duodenoscope. The baby scope 820 can be mechanically actuated or manipulated to cause the scope, and by extension the guide tube 810 and laser fiber 830 contained therein, to be displaced inside the working channel 844. For example, the baby scope 820 can be displaced so that it is extended through an opening 842 of the mother scope 840. In some embodiments, the guide tube 810 and the laser fiber disposed in the guide tube may each be independently actuated or manipulated to control their spatial displacement. Thus, the guide tube can be mechanically actuated to be extended and be advanced towards the target destination (e.g., proximate to the target area that is to be irradiated with radiation emitted from the laser fiber 830). In some embodiments, once the guide tube reaches its destination, the baby scope 820 can be actuated to cause it to be advanced from a position of inside the working channel 844 of the mother scope 840, along the extended guide tube 810, until the baby scope 820 reaches a position approximate to the position where the distal tip of the guide tube 810 is located. At that point, the laser fiber 830 may be actuated to be extended through the open end of the guide tube so that the distal end of the laser fiber protrudes from the guide tube and is positioned to irradiate the target area. The radiation source (not shown) can then be activated, resulting in radiation being coupled into the laser fiber and directed via the laser fiber to the fiber's end, whereupon the radiation is emitted and applied to the target area.

To actuate any of the various elements of the apparatus 800, including the guide tube 810, the baby scope 820, the laser fiber 830 and the mother scope 840, one or more actuation mechanisms may be used. For example, in some embodiments, the baby scope 820 can be caused to advance using an actuation mechanism implemented using, for example, an assembly of gears mechanically coupled to the baby scope such that upon activation of the assembly of gears the baby scope is spatially displaced. Under those circumstances, actuation of such a gear assembly can be controlled using a user interface (e.g., an interface that includes button, knobs, rotation wheels, etc.), and be controlled through the operator (e.g., a surgeon) of the apparatus 800. Such a gear assembly, or any other implementation of an actuation mechanism to actuate the baby scope 820, may be powered by the operator him/herself (e.g., by manual transfer of power through a rotation wheel to the gear assembly) or it may be powered by a power source coupled to the apparatus 800 (e.g., a motor). The other elements of the apparatus 800 may be similarly operated. In some embodiments, the operator may manually push and otherwise manipulate the baby scope.

As further shown in FIG. 8, to enable manipulation of the elements of the apparatus 800 to cause one or more of the elements to be deployed and to advance to their target destinations, the mother scope 840 includes steering mechanisms and components such as, for example, an elevator tooth 846. In some embodiments, the elevator tooth 846 is a pivotable inner projection to nudge the baby scope 820 and/or the guide tube 810 in a direction towards the opening 842 of the mother scope 840 to cause the guide tube 810 and/or the baby scope 820 to be deployed outside the mother scope 840. Particularly, when the elevator tooth 846 is controlled to pivot it to a position where it is configured to contact an advancing guide tube (or the baby scope 820) to nudge it towards the opening 842, the guide tube will be directed towards the opening 842 and be extended towards its destination position. Because the guide tube 810 is relatively flexible, contact between the elevator tooth 846 and the guide tube 810 generally does not compromise the structural integrity of the guide tube and does not result in damage to the guide tube. When the guide tube 810 has reached its final destination, the elevator tooth 846 may be actuated to pivot it to a lying position (e.g., so that it is substantially lying on the inner surface of the working channel 844 of the mother scope 840). The baby scope can then be manipulated to be advanced along the deployed guide tube, and can thus be advanced to its destination position without having to use the elevator tooth or some other steering or guiding mechanism that could potentially damage the relatively rigid and fragile baby scope 820.

Referring to FIG. 9, a partial view of an apparatus 900 that includes, among other things, a mother scope 940 and a guide tube 910, is shown. The apparatus 900 may be similar to the apparatus 800 depicted in FIG. 8. Apparatus 900 is shown, in FIG. 9, having the guide tube 910 deployed. Specifically, the hollow guide tube 910 is actuated to cause it to be advanced towards the target destination. For example, in some embodiments, an elevator tooth projecting from the surface of the working channel of the mother scope (as more particularly shown in FIG. 8) directs, or nudges, the guide tube through the opening 942 of the mother scope. As more of the guide tube is released, the guide tube advances towards the target destination. To ensure that the end of the guide tube 910 maintains the overall direction towards which it is supposed to be advancing, steering mechanisms can be used to control the direction of the guide tube. For example, the elevator tooth (similar to the elevator tooth 846 shown in FIG. 8) can be pivoted to various positions to control the direction of the advancing guide tube. Thus, if it becomes necessary to cause the guide tube to move more to the left side (the left side as shown in FIG. 9), the elevator tooth can be pivoted to a position that is farther away from the surface of the working channel of the mother scope (i.e., rotating the elevator tooth counterclockwise to increase the angle between the tooth and the surface of the working channel), to cause the guide tube to be pushed to the left hand side. During the deployment and advancement of the hollow guide tube 910, the laser fiber residing within the guide-tube extends out of the mother scope, but remains retracted from the distal end of the guide-tube to enable the guide-tube to retain its flexibility so that the guide-tube may more easily advance to its target destination. By keeping the end of the laser fiber retracted inside the guide tube, the laser fiber (or any other instrument that is held in the guide tube) is also protected from damage that may be caused during the deployment of the guide tube 910. Further, maintaining the instrument (be it a laser fiber or another type of instrument) within the guide tube 910 also protects the areas around the guide tube (tissue and organs) from injury from the potentially abrasive and sharp instrument inside the guide tube 910.

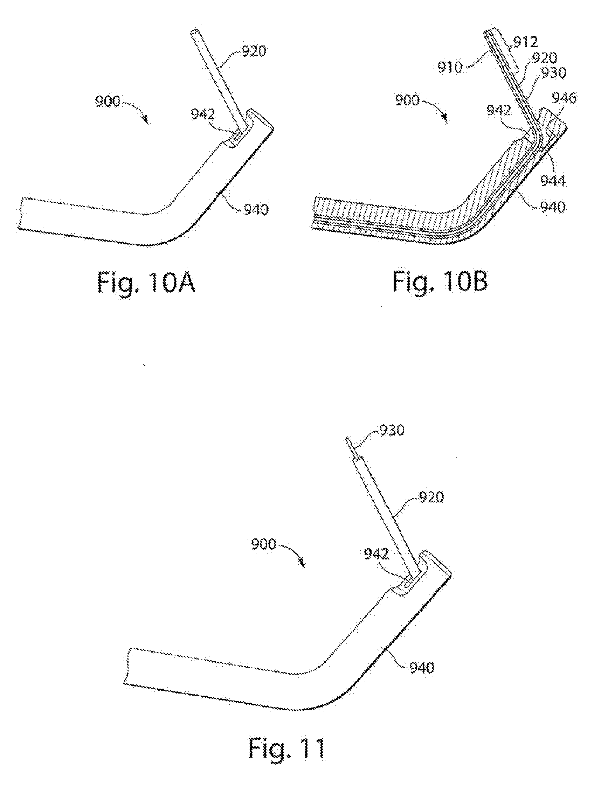

Referring to FIGS. 10A-B, diagrams depicting the apparatus 900 of FIG. 9 after the guide-tube has been deployed proximate to the target area to be treated are shown. Specifically, as shown in the perspective diagram of FIG. 10A, once the guide tube 910 has reached its destination position, the baby scope 920 may be pushed (e.g., by the operator) from its position within the working channel 944 (shown in cross-sectional diagram of FIG. 10B) to extend from the mother scope 940 and slide over the guide-tube 910 until the baby scope 920 reaches approximately the distal end of the guide-tube 910 and covers the guide-tube 910 substantially entirely. It should be noted that the end of the baby scope can generally be deflected (maneuvered) in various directions as controlled by the operator.

As can further be seen from FIGS. 10A and 10B, as the baby scope 920 advances along the flexible guide tube 910, the guide tube 910 is received within the inner channel of the baby scope 920. As further shown in FIG. 10B, after the baby scope 920 is advanced to cover substantially entirely the guide tube 910, in some embodiments, the instrument contained with the guide tube, in this case the laser fiber 930, is still disposed within the guide tube such that it does not occupy an inner lumen 912 defined by the end section of the guide tube 910.

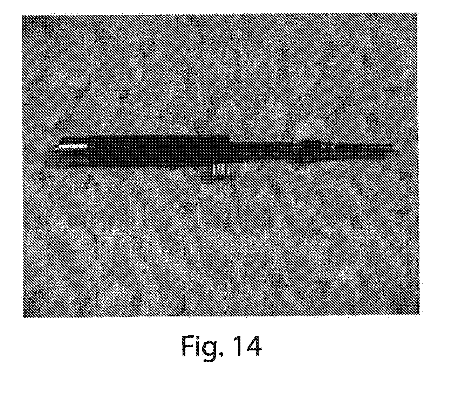

Referring to FIG. 11, a diagram depicting the apparatus 900 of FIGS. 9 and 10 at a time after the baby scope has been deployed proximate to the target area is shown. Once the baby scope has been extended to the distal end of the guide-tube 910, the laser fiber 930, which up until now was disposed entirely within the guide tube 910, is extended or slid out from the interior of the guide-tube 910. To extend or displace the laser fiber 930, an operator may manually push the laser fiber. Alternatively and/or additionally, in some embodiments, a laser shifter, such as the one depicted in the photograph of FIG. 14, may facilitate displacement of the laser fiber to advance it along the interior of the guide tube. Once the laser fiber has been extended out of the guide-tube/baby scope assembly, a laser source (not shown) may be activated to deliver radiation through the laser fiber to irradiate the target area to be treated. For example, in some embodiments, the radiation emitted from the tip of the laser fiber 930 is applied to an obstruction, such as a bile stone, to break up that obstruction.

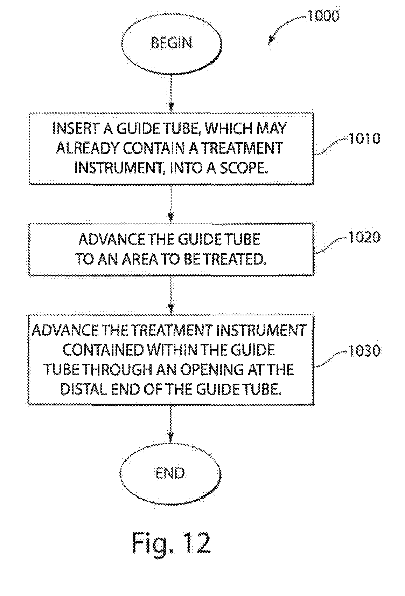

Referring to FIG. 12, a flowchart of a procedure 1000 performed using, for example, an apparatus such as the apparatus 900 illustrated in FIGS. 9-11, is shown. Initially, a hollow guide tube, such as any of the guide tubes depicted in FIGS. 5-11, is inserted 1010 into a scope (a scope such as any of the baby scopes depicted in FIGS. 5-11). In some embodiments, the hollow guide tube already contains an instrument, such as a laser fiber configured to emit radiation from its end (e.g., a modifiable laser structure such as the structure 200 depicted in FIG. 2 and described herein). The instrument, in this case, the laser fiber, may be disposed such that it does not extend all the way to the opening at the tip of the guide tube. Under such circumstances, the guide tube includes an end section not occupied by any part of the instrument. Such a configuration provides the end section of the guide tube with additional flexibility to enable it to be bent, contorted and/or otherwise manipulated.

In some embodiments, the baby scope into which the guide tube has been inserted is inserted into a working channel of another, larger scope (mother scope), such as a duodenoscope. The mother scope can be directed to a location proximate to where the guide tube it to be deployed (e.g., near the opening of a bile duct).

The hollow guide tube placed inside the baby scope is advanced 1020 to an area to be treated. To advance the guide tube, a displacement/actuation mechanism that causes the guide tube to be advanced may be operated. For example, in some embodiments, in response to a user pressing an activation button, a spool, powered by a motor, begins to rotate and to release sections of the guide tube that are rolled on the spool. In some embodiments, steering mechanisms, for example, a pivotable elevator tooth projecting from the inner surface of the working channel of the mother scope can be actuated (e.g., pivoted clockwise or counterclockwise) to push, or nudge, the guide tube towards an opening of the mother scope, and to further steer the guide tube (in response to the movement of the pivotable elevator tooth) to be directed towards the destination location for the tip of the guide tube. Other types of steering and/or displacement mechanisms to control the movement of the guide tube may be used.

After releasing the guide tube so that it extends from the baby scope within which it was disposed, the baby scope is slid along the guide tube. Thus, the baby scope tracks the guide tube so that the guide tube is received back into the inner channel of the baby scope. As a result, to advance the baby scope to the destination location, it is not necessary to operate any steering mechanism, such as the pivotable elevator tooth, and accordingly, the risk that the baby scope might be damaged during its deployment is reduced.

After the guide tube and/or the baby scope that was advanced along the released guide tube have reached the destination location near the target area to be treated, the instrument (e.g., the laser fiber) contained within the guide tube is advanced 1030 through an opening at the distal end of the guide tube such that at least part of the treatment instrument extends outside the distal end of guide tube and/or the baby scope. With the instrument extending from outside the guide tube, the instrument, can perform its operations on the area to be treated. For example, in circumstances in which the instrument is a laser fiber, radiation generated by a radiation source and coupled to the laser fiber is emitted through the distal tip of the laser fiber to irradiate the target area to be treated.

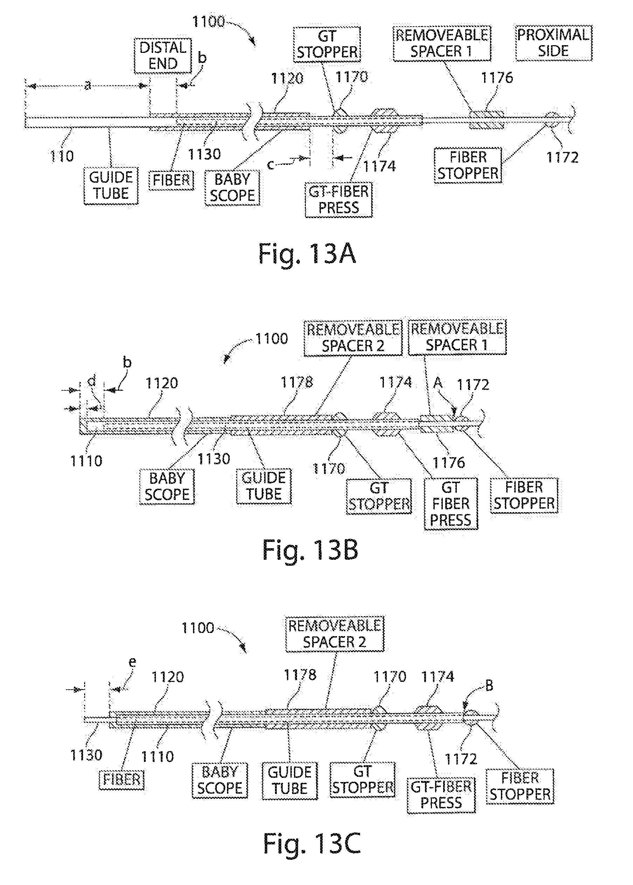

The apparatus that includes a baby scope containing a guide tube in which an instrument, such a laser fiber, may be disposed, may be used with a variety of applications, including medical applications, industrial applications, etc., and may be used to perform a number of functions in its various configurations, including, for example, cutting, ablating, etc. Referring to FIGS. 13A-G, diagrams of an apparatus 1100 used to perform a medical procedure is shown. The apparatus 1100, which may be similar to any of apparatus 500, 600, 700, 800 and 900 described herein, may be utilized to perform a bile duct obstruction removal procedure (similar to the procedure described in relation to FIGS. 4A and 4B, except that in the procedure depicted in relation to FIGS. 13A-G, a guide tube based apparatus may be utilized). Other types of medical procedures in which an apparatus such as the apparatus 1100 can be directed through tubular anatomical structures of the body to operate the instrument contained within a guide tube disposed within a baby scope include procedures to open strictures in the upper or lower airways (trachea and bronchi), procedures performed at the urinary tract that include opening strictures in the ureters to subsequently break urinary stones, etc.

As shown in FIG. 13A, a treatment instrument, such as a laser fiber 1130, is inserted through the proximal end of the guide tube 1110 and into a bore (channel) of the guide tube. The instrument, in this case the laser fiber, is generally advanced inside the bore of the guide tube 1110 until the laser fiber is some distance away from the open distal end of the guide tube 1110. That is, the inner volume at the guide tube's distal end will not be occupied by any part of the instrument inserted into the bore of the guide tube 1110. Thus, the distal end of the guide tube 1110 will be flexible relative to the rigid remaining part of the guide tube containing the instrument.

As further shown in FIG. 13A, discernable markers to enable an operator to ascertain the relative positions between the various elements (e.g., the guide tube, the fiber) of the apparatus may be included. Specifically, when using a guide tube with a fiber inserted into it, and passing that arrangement through a baby scope 1120, there is typically no restriction imposed on the relative position of each element with respect to the others. However, it is generally important that the guide tube or the sharp edged fiber will not protrude unintentionally from the baby scope inside the CBD. Therefore, both the fiber and the guide tube should have discernable markers on them to enable a physician to know where the fiber and guide tube tips are positioned with respect to the baby scope, as well as relative to each other. Accordingly, in some implementations, visual or otherwise discernable markers may be added to elements of the structure (e.g., the fiber, the guide tube, etc.) to enable a determination of the relative positions of the elements and also prevent unintentional movement between them. Such markers may include threaded stoppers, such as a guide tube stopper 1170, placed over the guide tube 1110, and the fiber stopper 1172, placed over the fiber 1130. In addition to marking the positions of the fiber and/or guide tube, these stoppers also limit the movement of the fiber and guide tube. Other types of markers include devices/structures to set the relative positions between the elements by pressing them one against the other, e.g., clamping mechanisms such as the guide tube fiber press 1174. In some embodiments, the markers may also include spacers, such as a spacer 1176, having defined lengths and determined distances between them. Such spacers can subsequently be removed, replaced or reset to define or set another pre-determined relative position between the elements.

In FIG. 13A the apparatus 1100 is arranged in the so-called "Guide Wire" configuration. This configuration is in effect until the guide tube is positioned within the CBD. In the "Guide Wire" configuration, a maximal distance "a" is determined by the position of the guide tube stopper 1170 on the guide tube 1110. In this configuration, it is possible to push the guide tube/fiber arrangement to cause the arrangement to extend beyond the baby scope's distal end by controlling the distance "c". When the distance c is equal to 0, the guide tube has reached its farthest point relative to the baby-scope 1120. As further shown in FIG. 13A, the relative position between the guide tube and the fiber is established using the GT-Fiber press 1174.

Referring to FIG. 13B, a schematic diagram of the apparatus 1100 when the arrangement of the guide tube/fiber/baby scope is ready to be placed near a target area (e.g., an obstruction such as a stone) is shown. As depicted in FIG. 13B, in this configuration the baby scope slides over the guide tube until it substantially covers the guide tube 1110. Once the baby scope is advanced to cover substantially entirely the guide tube 1110, the fiber 1130 is pushed until the fiber stopper 1172 reaches point A where it touches the removable spacer 1176. As shown, in that position the distal end of the fiber 1130 is at a distance of "b-d" from the distal end of the guide tube 1110. This distance is based on the lengths of the guide tube 1110, the fiber 1130 and the removable spacer 1176. As further shown in FIG. 13B, in some embodiments, a removable spacer 1178 is placed so as to fix the distance "d" between the guide tube and the baby scope's distal end. The relative position between the guide tube and the fiber is established using the guide tube/fiber press 1174.

Referring to FIG. 13C, a schematic diagram of the apparatus 1100 when the arrangement of the guide tube/fiber/baby scope is in a "firing" configuration (e.g., laser radiation is about to be emitted at an obstruction via the fiber) is shown. In this configuration, the removable spacer 1176 shown in FIGS. 13A and 13B is removed (the removable spacer 1178 remains in place). Having removed the spacer 1176, the fiber 1110 can now be further pushed (i.e., beyond the point A illustrated in FIG. 13B). Thus, as shown in the diagram of FIG. 13C, the fiber 1130 is pushed so that, with respect to the proximal end of the fiber, the fiber reaches a point B. With respect to the distal end of the fiber, the fiber has been pushed so that it now extends a distance "e" from the baby scope distal end. In that position, radiation delivered through the fiber 1110 can be emitted at the obstruction to be removed.

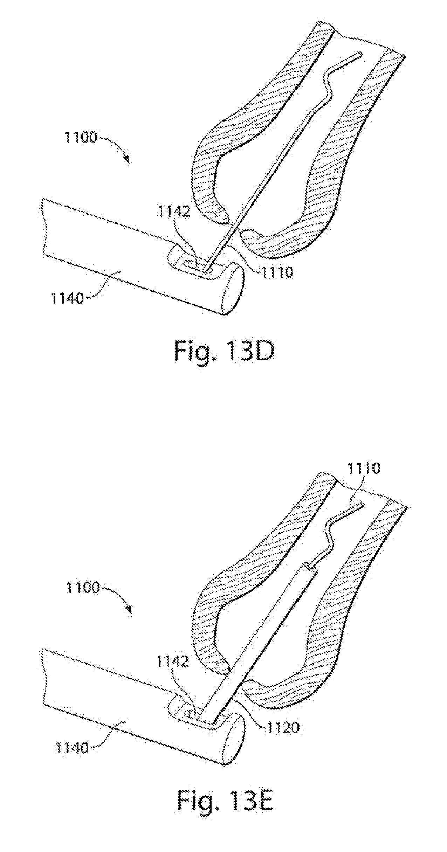

Referring to FIG. 13D, a diagram of the apparatus deploying the guide tube (e.g., prior to being placed in the configurations depicted in the schematic diagrams of FIGS. 13A and 13B) is shown. As described herein, prior to being advanced to a position near the obstruction to be removed, the baby scope 1120 (schematically depicted in FIGS. 13A-C) fitted with the guide tube 1110 containing the laser fiber 1130 is placed inside a working channel of another scope 1140, e.g., duodenoscope (the mother scope). The arrangement of the baby scope, guide tube and laser fiber contained within the guide tube is then advanced to a position proximate to an opening 1142 of the mother scope.

The apparatus 1100, including the baby scope placed inside the mother scope with the baby scope is guided to the area near the target area to be treated (in this case, the papilla near the opening of the common bile duct). In some embodiments, it may be necessary to open the bile duct to provide access to the obstruction to be removed. In some embodiments, the procedure to open the bile duct may be performed using, for example, an electrical wire and/or a laser-based instrument, such as a modifiable laser fiber 200 described herein. Other instruments to open or enlarge the opening of the bile duct may be used.

As shown in FIG. 13D, when the mother scope 1140 reaches its destination location, the guide tube 1110 is actuated to cause the guide tube 1110 to extend from the end section of the baby scope 1120, through the opening 1142 of the mother scope 1140, and into the bile duct towards the location of the obstruction that is to be removed. In some embodiments, a pivotable elevator tooth (such as the one depicted in FIG. 10B) is actuated to cause it to pivot upwards or downwards to thus cause the advancing guide tube to be bent to enable positioning it opposite the opening of the bile duct. Other steering/guidance mechanisms may be employed to control the advancement of the guide tube 1110.

Referring to FIG. 13E, once the guide tube 1110 has been substantially deployed to its destination position, the baby scope 1120 is advanced along the guide tube 1110. In other words, the baby scope 1120 tracks the guide tube 1110, causing the guide tube 1110 to be received within the inner channel of the baby scope 1120. To cause the baby scope 1120 to be displaced towards the position occupied by the tip of the deployed guide tube 1110, the operator (e.g., the physician) pushes, for example, the baby scope to cause the baby scope to advance along the guide tube 1110. However, use of a steering mechanism to operate on the baby scope, such as the elevator tooth used to steer the guide tube 1110, is not required because the destination to which the baby scope needs to be advances is already defined by the guide tube 1110. The baby scope merely needs to be pushed or to be otherwise moved to track the guide tube without a steering mechanism(s) being applied to the baby scope 1120 in a way that could damage the scope 1120. In some embodiments, when the baby scope 1120 is being advanced along the guide tube, the elevator tooth may be actuated to its lying position so that the elevator tooth does not exert any force on the baby scope that could damage the baby scope 1120. The baby scope distal part may have a steering mechanism of its own which may be used once it is already positioned within the bile duct to allow control of direction, primarily at bifurcations points (i.e., at points in which two or more tubes extend from the tube the scope is currently traversing, thus requiring the operator to determine which tubular "branch'" to steer the scope to).

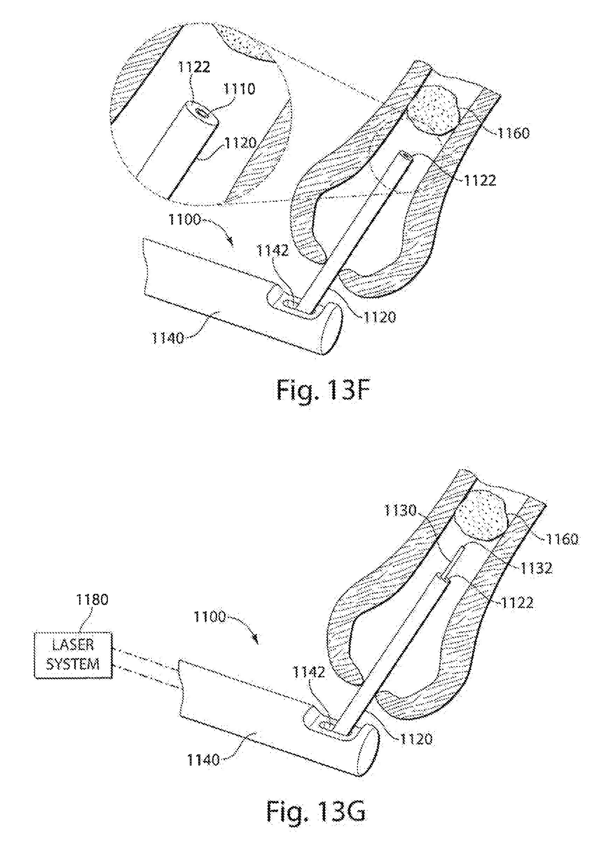

As shown in FIG. 13F, the baby scope 1120 continues to be advanced along the deployed section of the guide tube 1110 until the baby scope 1120 covers the entire guide tube 1110 (i.e., until substantially the entire guide tube is received within the inner channel of the baby scope 1120), As also shown in FIG. 13F, the distal end 1122 of the baby scope 1120 (now covering substantially entirely the guide tube 1110) is positioned proximate to an obstruction (e.g., a stone 1160), that is to be operated upon by the apparatus 1100.

Referring to FIG. 13G, after the baby scope is advanced to substantially cover the guide tube 1110, the laser fiber 1130 is actuated to cause it to be extended from the opening of the hollow guide tube 1110. As noted, the laser fiber may be moved by the operator. The protruding laser fiber 1130 has its end directed substantially at the direction of the obstruction (the stone 1160 in FIG. 13G), and upon activation of a laser source 1180 coupled to the laser fiber, laser radiation is emitted from the end 1132 of the laser fiber 1130 and applied to the stone 1160. The radiation applied has a suitable wavelength and sufficient fluence to break-up the stone, thus causing the obstruction to be released or all together removed. Suitable laser systems that may be used include, for example, Er:YAG laser systems, Ho:YAG laser systems and Nd:YAG laser systems. Other types of laser systems may also be used.

In some embodiments, upon completion of the laser irradiation operation, the laser fiber 1130 may be retracted into the hollow guide tube 1110 inside the baby scope 1120, and the baby scope 1120 may then be retracted and removed from the working channel of the mother scope 1140. Subsequently, another scope containing another instrument may be passed through the working channel of the mother scope 1140 and be extended to the opening 1142 of the scope 1140, whereupon other operations may be performed in the area where the opening 1142 of the scope 1140 is located. In some embodiments, the guide-tube with the fiber may be withdrawn and other instruments (e.g., a basket, a balloon, etc.) may subsequently be inserted through the working channel of the baby scope which has been left in the CBD.

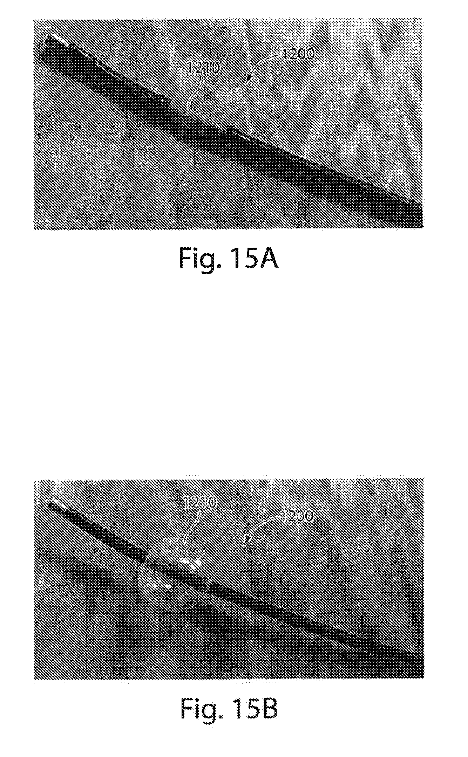

Referring to FIGS. 15A-B, photographs of a scope-based device (e.g., endoscope) 1200 that includes a positioning balloon 1210 in a deflated and inflated state, respectively, are shown. The endoscope 1200 in FIGS 15A-B may be similar in structure and functionality to any of the scopes described herein (e.g., any of the mother scopes and/or baby scopes), except that the endoscope 1200 includes a balloon configured to position the scope (e.g., center the scope) within the tubular structures (organs) through which the scope 1200 passes.

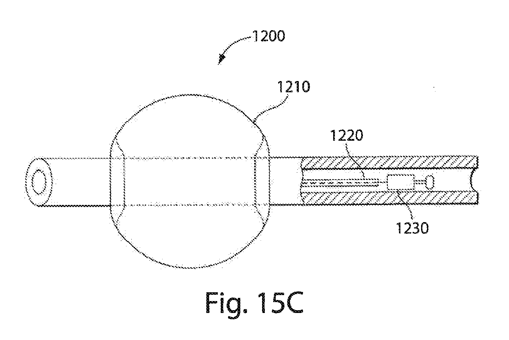

Particularly, and with reference to FIG. 15C, showing a schematic diagram of an implementation of the scope 1200 with the balloon 1210 inflated, the scope 1200 may include, in some embodiments, a tube/conduit 1220 coupled to an opening in the balloon 1210. The tube 1220 is generally disposed within the interior of the scope 1200. The tube 1220 is configured to deliver fluid (e.g., water, various gases) and/or remove fluid from the balloon 1210. In some embodiments, the tube may be connected, at the tube's other end, to a syringe 1230 containing fluid that is delivered to the balloon upon actuation of the syringe. The syringe may be attached to the proximal user end of the endoscope that can be manipulated by the operator.

With reference again to FIG. 15A, in its deflated configuration the scope 1200 can be passed through tubular structures in a patient's body en route to the target area that is to be viewed or treated by the scope 1200. Once the scope 1200 has reached a particular position (either the final destination or some intermediary point where some functionality of the scope is to be used (e.g., viewing the area where the scope 1200 is located), fluid or gas is directed into the balloon 1210 to cause the balloon to inflate. The inflated balloon surrounds at least part of the scope (e.g., when inflated, a section of the tubular scope may be contained within the inflated balloon). Inflating the balloon causes several effects, including: a) centering the scope 1200 within a tube like organ (the CBD, bronchus, ureter, etc.), and b) stabilizing the endoscope opposite the target to be treated (stone, obstruction, etc.) such that the image captured by an endoscope is stable throughout a procedure to be performed (a procedure such as the one described, for example, in relation to FIGS 13A-G), so that movement that may be caused by the treatment tool (e.g., a laser fiber irradiating an obstruction with laser radiation) is avoided. Upon___ completion of the procedure, the fluid/gas contained in the inflateable balloon may be withdrawn by, for example, using the syringe. When the fluid/gas is sufficiently withdrawn, the scope 1200 may be maneuvered again through the tubular structures in the body.

### OTHER EMBODIMENTS

A number of embodiments of the invention have been described. Nevertheless, it will be understood that various modifications may be made without departing from the spirit and scope of the invention. Accordingly, other embodiments are within the scope of the following claims.

## Claims

1. A fiber structure comprising:
an energy-transmitting fiber; and
a cover surrounding the energy-transmitting fiber, the energy transmitting fiber including an end that is substantially flush with the cover such that the fiber structure can be passed through an inner channel of a device to receive the fiber structure.

2. The fiber structure of claim 1, wherein the fiber structure that can be passed through the inner channel of a device is configured to be guided naturally through the inner channel of the device without using guiding mechanisms, the guiding mechanisms including a guide wire.

3. The fiber structure of claim 1 or claim 2, wherein the energy transmitting fiber is an optical fiber.

4. The fiber structure of any preceding claim, wherein the energy transmitting fiber is optically coupleable to a laser subsystem.

5. The fiber structure of any preceding claim, wherein the fiber structure configured to be passed through the inner channel of a device is configured to be passed through a working channel of a scope-based device, the scope-based device including one of: a single scope-based system, and a mother scope-baby scope system.

6. An apparatus comprising:
a device including an inner channel; and
a fiber structure according to any of claims 1 to 3, configured to be passed through the inner channel of the device.

7. The apparatus of claim 6, wherein the device including the inner channel comprises a scope-based device.

8. The apparatus of claim 7, wherein the fiber is no more than about 2 mm in diameter smaller than a working channel of the scope-based device.

9. The apparatus of claim 7 or claim 8, wherein the scope-based device includes a mother scope-baby scope endoscopic system comprising a mother scope including a working channel, and a baby scope fitting within the working channel of the mother scope,
and optionally wherein the mother scope includes a duodenoscope
and further optionally, wherein the baby scope includes a choledocoscope.

10. The apparatus of claim 7 or claim 8, wherein the scope-based device is a single scope-based system.

11. The apparatus of any of claims 6 to 10, further comprising:
a laser subsystem optically coupleable to the energy-transmitting fiber.

12. A method comprising:
inserting a fiber structure comprising an energy-transmitting fiber and a cover surrounding the energy-transmitting fiber into a device including an inner channel, the energy-transmitting fiber including an end that is substantially flush with the cover such that the fiber structure can be passed through the inner channel of the device; and
advancing the fiber structure through the inner channel of the device.

13. The method of claim 12, wherein the fiber structure that can be passed through the inner channel of the device is configured to be guided naturally through the inner channel of the device without using guiding mechanisms, the guiding mechanisms including a guide wire.

14. The method of claim 12 or claim 13, wherein the device comprises a scope based device including one of: a single scope-based system, and a mother scope-baby scope system
and, wherein if the device is a mother scope-baby scope system, the method optionally further comprises:
inserting a mother scope and a baby scope of the mother scope-baby scope system into a patient's body;
guiding the baby scope so that the baby scope's end is pointing in an appropriate direction for application of energy to achieve a desired therapeutic purpose;
directing the fiber structure including the fiber with the substantially flush end through the baby scope's working channel without using a guide wire; and
coupling optical energy through the fiber, the optical energy being emitted from the substantially flush end of the fiber towards a target,

15. The method of any of claims 12 to 14, further comprising:
optically coupling a laser subsystem to the energy-transmitting fiber
